# EUROPEAN PATENT APPLICATION

(11) **EP 4 567 045 A1**
(43) Date of publication of application: **11.06.2025**
(21) Application number: 23849474.4
(22) Date of filing: 02.08.2023
(51) Int. Cl.: C07K 16/28, A61K 39/395

(54) **ANTI-DDR2 NANO-ANTIBODY AND USE THEREOF**

(30) Priority: 03.08.2022 CN 202210925764
(71) Applicant: Fibro-Biomed Technology (Guangzhou) Company, Ltd., Guangzhou, Guangdong 510700 (CN)
(72) Inventor: SU, Jin, Guangzhou, Guangdong 510700 (CN); WEI, Xinru, Guangzhou, Guangdong 510700 (CN); LAI, Yunxin, Guangzhou, Guangdong 510700 (CN); ZHANG, Ping, Guangzhou, Guangdong 510700 (CN); SHI, Miao, Guangzhou, Guangdong 510700 (CN); JIANG, Jinjun, Guangzhou, Guangdong 510700 (CN); HUANG, Xihui, Guangzhou, Guangdong 510700 (CN)
(74) Representative: Patent 42
(86) International application number: PCT/CN2023/110789
(87) International publication number: WO 2024/027770

(57) **Abstract**

The present invention relates to anti-DDR2 antibodies. More specifically, the present invention relates to nano-antibodies binding to DDR2 and various derivatives of the nano-antibodies. The present invention also relates to pharmaceutical compositions comprising these antibodies and derivatives and pharmaceutical uses thereof. The anti-DDR2 nano-antibodies of the present invention have strong binding activity and can be used for diagnosis, prevention and/or treatment of diseases mediated by abnormal DDR2 expression (such as cancer and inflammatory diseases), as well as imaging of cells expressing DDR2, such as biopsy navigation and intraoperative navigation.

## Description

### TECHNICAL FIELD

The present invention relates to anti-DDR2 antibodies. More specifically, the present invention relates to nano-antibodies binding to DDR2 and various derivatives of the nano-antibodies. The present invention also relates to pharmaceutical compositions comprising these antibodies and derivatives and pharmaceutical uses thereof.

### BACKGROUND

Discoidin domain receptor 2 is a receptor tyrosine kinase (RTK), which uses extracellular matrix protein collagen as its ligand. In addition to its kinase function, DDR2 also promotes cell adhesion by activating β1-integrin. The unique function of DDR2 is to mediate the signal transmission of extracellular matrix into cells, so that the regulation of extracellular matrix is balanced, and participate in the regulation of cell growth, differentiation and metabolism. Although the activation of DDR2 by extracellular matrix collagen protein is necessary for normal development and tissue homeostasis, the abnormal activation of these receptors after injury or disease is harmful.

The role of DDR2 in the regulation of extracellular matrix indicates that its signal imbalance may cause cancer, including invasion and metastasis. It is reported that DDR2 is highly expressed at the forefront of many invasive breast tumor samples. In situ hybridization on adjacent sections of human ovarian or lung cancer showed that DDR2 was detected in stromal cells around the tumor. Therefore, DDR2 is considered to be crucial for the metastasis of many cancer cells (such as breast cancer cells), and it is also an important target for many other cancers (such as ovarian cancer, lung cancer, head and neck cancer, pancreatic cancer, etc.).

DDR2 is also considered to be an important target for inflammation such as arthritis (such as osteoarthritis, rheumatoid arthritis) and fibrosis (such as pulmonary fibrosis, liver cirrhosis, renal fibrosis or skin fibrosis). DDR2 is mainly expressed in interstitial cells such as fibroblasts, myofibroblasts and smooth muscle cells in kidney, skin, lung, heart and connective tissue. Many evidences show that the abnormal expression of DDR2 is related to a variety of disease processes, such as inflammation, liver fibrosis, renal fibrosis, pulmonary fibrosis, skin scar and atherosclerosis. In the study of mouse inflammation model, it was found that DDR2 expression was up-regulated in the knee joint of elderly rats. In the study of rat model of rheumatoid arthritis, it was found that the expression of DDR2 in synovial cells was up-regulated.

WO2019243431A1 discloses that in melanoma cell-derived xenograft models, targeting DDR2 through Imatinib enhances BRAF inhibitor anti-tumor efficacy, delays the onset of acquired resistance and counteracts targeted therapy-induced tumor fibrosis.

Therefore, the development of antibodies with high affinity targeting DDR2 has a wide range of practical significance for the imaging, diagnosis, prevention and treatment of cancer, inflammation, fibrosis and other diseases.

### SUMMARY

In a first aspect, the present invention provides a nano-antibody binding to DDR2, wherein the nano-antibody comprises a CDR1, a CDR2, and a CDR3 comprising or being the CDR1, CDR2, and CDR3 of a nano-antibody selected from the amino acid sequence of any one of SEQ ID NO: 4, SEQ ID NO: 8, SEQ ID NO: 12, SEQ ID NO: 16, and SEQ ID NO: 20, or an equivalent variant of each thereof.

In some embodiments, the CDR1, CDR1, and CDR3 are defined based on any one of the IMGT, Kabat, Chothia, Contact, or Martin definition scheme.

In some embodiments, the nano-antibody comprises a CDR1, a CDR2, and a CDR3, and
(a) the CDR1 comprises or is the sequence set forth in SEQ ID NO: 1 or an equivalent variant thereof, the CDR2 comprises or is the sequence set forth in SEQ ID NO: 2 or an equivalent variant thereof, the CDR3 comprises or is the sequence set forth in SEQ ID NO: 3 or an equivalent variant thereof;
(b) the CDR1 comprises or is the sequence set forth in SEQ ID NO: 5 or an equivalent variant thereof, the CDR2 comprises or is the sequence set forth in SEQ ID NO: 6 or an equivalent variant thereof, the CDR3 comprises or is the sequence set forth in SEQ ID NO: 7 or an equivalent variant thereof;
(c) the CDR1 comprises or is the sequence set forth in SEQ ID NO: 9 or an equivalent variant thereof, the CDR2 comprises or is the sequence set forth in SEQ ID NO: 10 or an equivalent variant thereof, the CDR3 comprises or is the sequence set forth in SEQ ID NO: 11 or an equivalent variant thereof;
(d) the CDR1 comprises or is the sequence set forth in SEQ ID NO: 13 or an equivalent variant thereof, the CDR2 comprises or is the sequence set forth in SEQ ID NO: 14 or an equivalent variant thereof, the CDR3 comprises or is the sequence set forth in SEQ ID NO: 15 or an equivalent variant thereof; or
(e) the CDR1 comprises or is the sequence set forth in SEQ ID NO: 17 or an equivalent variant thereof, the CDR2 comprises or is the sequence set forth in SEQ ID NO: 18 or an equivalent variant thereof, the CDR3 comprises or is the sequence set forth in SEQ ID NO: 19 or an equivalent variant thereof.

In some embodiments, the nano-antibody comprises or is the sequence selected from any one of SEQ ID NO: 4, SEQ ID NO: 8, SEQ ID NO: 12, SEQ ID NO: 16, and SEQ ID NO: 20, and an equivalent variant of each thereof.

In another aspect, the present invention provides a polynucleotide comprising a polynucleotide encoding any one of the antibodies described above. In another aspect, the present invention provides a vector comprising the polynucleotide described above. In another aspect, the present invention further provides a non-human host cell comprising the vector described above. In addition, the present invention further provides a cell line for generating the antibody of the present invention, and a method for preparing the antibody by culturing an antibody-producing cell line.

In a second aspect, the present invention provides a chimeric antigen receptor, comprising an extracellular domain capable of binding to an antigen, a transmembrane domain, and an intracellular domain, wherein the extracellular domain capable of binding to an antigen comprises any one of the nano-antibodies according to the first aspect. In another aspect, the present invention provides a polynucleotide comprising a polynucleotide encoding the chimeric antigen receptor described above. In another aspect, the present invention provides a vector comprising the polynucleotide described above. In another aspect, the present invention provides a modified immune cell expressing the chimeric antigen receptor described above.

In a third aspect, the present invention provides a bispecific antibody, comprising a first binding moiety binding to a first antigen and a second binding moiety binding to a second antigen, wherein the first binding moiety comprises any one of the nano-antibodies according to the first aspect.

In a fourth aspect, the present invention provides an antibody-drug conjugate, comprising an antibody targeting DDR2, a drug, and a linker connecting the antibody and the drug, wherein the antibody targeting DDR2 comprises or is any one of the nano-antibodies according to the first aspect.

In a fifth aspect, the present invention provides an siRNA conjugate targeting a DDR2 positive cell, comprising: (i) an antibody targeting DDR2, which comprises or is any one of the nano-antibodies according to the first aspect, (ii) an siRNA that inhibits the expression of a survival gene in a DDR2 positive cell, and (iii) a linker positioned between the nano-antibody and the siRNA.

In a sixth aspect, the present invention provides a pharmaceutical composition comprising a therapeutically effective amount of any one of the antibody, polynucleotide, vector, host cell, immune cell, and/or conjugate described above, and a pharmaceutically acceptable carrier.

In a seventh aspect, the present invention provides use of any one of the antibody, polynucleotide, vector, host cell, immune cell, or conjugate described above in the manufacture of a medicament for treating and/or preventing a disease mediated by abnormal DDR2 expression.

In an eighth aspect, the present invention provides any one of the antibody, polynucleotide, vector, host cell, immune cell, or conjugate described above for use in treating and/or preventing a disease mediated by abnormal DDR2 expression.

In a ninth aspect, the present invention provides a method for treating and/or preventing a disease mediated by abnormal DDR2 expression, comprising administering to a subject an effective amount of any one of the antibody, polynucleotide, vector, host cell, immune cell, or conjugate described above.

In a tenth aspect, the present invention provides a reagent for imaging and/or diagnosis, comprising any one of the nano-antibodies according to the first aspect and a detectable label connected with the nano-antibody.

In an eleventh aspect, the present invention provides use of the reagent for imaging and/or diagnosis according to the tenth aspect in the manufacture of a kit for the diagnosis of a disease mediated by abnormal DDR2 expression in a subject.

In a twelfth aspect, the present invention provides a reagent for imaging and/or diagnosis according to the tenth aspect for use in the diagnosis of a disease mediated by abnormal DDR2 expression in a subject.

In a thirteenth aspect, the present invention provides a method for the diagnosis of a disease mediated by abnormal DDR2 expression in a subject, comprising administering to the subject an effective amount of the reagent for imaging and/or diagnosis according to the tenth aspect. In some embodiments, the method comprises: administering to the subject an effective amount of the reagent for imaging and/or diagnosis according to the tenth aspect; detecting and reading a signal generated by the reagent for imaging and/or diagnosis; and determining that the subject has or is at risk of having a disease mediated by abnormal DDR2 expression based on the strength of the signal.

In a fourteenth aspect, the present invention provides a method for treating a disease mediated by abnormal DDR2 expression in a subject, comprising: (a) administering to the subject an effective amount of any one of the reagent for imaging and/or diagnosis described above; (b) detecting and reading a signal generated by the reagent for imaging and/or diagnosis; (c) determining whether the signal exceeds a predetermined threshold, and and when the signal exceeds the predetermined threshold, determining that the subject is suffering from a disease mediated by abnormal DDR2 expression, preferablly wherein the threshold is a median level from a subject not suffering from the disease; and (e) administering to the subject determined to be suffering from the disease a therapy that reduces DDR2 activity and/or expression, such as anticancer therapy or anti-inflammatory therapy, such as anti-fibrosis therapy. In a preferred embodiment, the anti-fibrosis therapy is Pirfenidone and/or Nintedanib.

In the above related aspects, the disease mediated by abnormal DDR2 expression comprises cancer and inflammatory disease. In some embodiments, the cancer comprises but is not limited to melanoma, breast cancer, colon cancer, kidney cancer, liver cancer, lung cancer, head and neck cancer, ovarian cancer, pancreatic cancer, prostate cancer, uterine cancer, cervical cancer, bladder cancer, gastric cancer, and skin cancer. In some embodiments, the inflammatory disease comprises but is not limited to rheumatoid arthritis, osteoarthritis, skin scar, atherosclerosis, retinal vasculopathy, and fibrosis. The fibrosis comprises but is not limited to pulmonary fibrosis (especially idiopathic pulmonary fibrosis), liver fibrosis, liver cirrhosis, skin fibrosis, renal fibrosis, pancreatic fibrosis, systemic sclerosis, cardiac fibrosis, and macular degeneration.

The anti-DDR2 nano-antibodies of the present invention have strong binding activity and can be used for diagnosis, prevention and/or treatment of diseases mediated by abnormal DDR2 expression (such as cancer and inflammatory diseases), as well as imaging of cells expressing DDR2, such as biopsy navigation and intraoperative navigation.

Other aspects and advantages of the present invention are apparent from the following detailed description of the invention.

### BRIEF DESCRIPTION OF THE DRAWINGS

**FIG. 1****:** SDS-PAGE results of the expression and purification of DDR2 nano-antibodies.
**FIG. 2****:** ELISA validation results of DDR2 nano-antibodies.
**FIG.** 3: Flow cytometry results of DDR2 nano-antibodies.
**FIG. 4****:** Immunofluorescence images of DDR2 nano-antibodies.
**FIG. 5****:** Affinity constant plots of DDR2 nano-antibodies and extracellular segment of DDR2 antigen.
**FIG. 6****:** In vitro imaging results of mouse lung tissue with DDR2 nano-antibody.
**FIG. 7****:** In vitro imaging results of human lung tissue with DDR2 nano-antibody.
**FIG. 8****:** In vitro imaging results of porcine and rabbit lung tissues with DDR2 nano-antibody.
**FIG. 9****:** In vitro retinal imaging results with DDR2 nano-antibody.
**FIG. 10****:** In vivo imaging results with DDR2 nano-antibody.
**FIG. 11****:** Western blot (WB) images of nano-antibody 1A12 inhibiting collagen Collagen1 induced DDR2 protein phosphorylation.
**FIG. 12****:** Statistical graph of nano-antibody 1A12 inhibiting collagen Collagen1 induced DDR2 protein phosphorylation.
**FIG. 13****:** DNA electrophoresis image of plasmid digestion identification of anti-DDR2 nano-antibody expression.
**FIG. 14****:** SDS-PAGE electrophoresis staining of purified anti-DDR2 nano-antibody.
**FIG. 15****:** Western blot image of purified anti-DDR2 nano-antibody.
**FIG. 16****:** Flow cytometry analysis of anti-DDR2 nano-antibody activity.
**FIG. 17****:** Western blot image of anti-DDR2 nano-antibody inhibiting collagen induced DDR2 protein phosphorylation.
**FIG. 18****:** Schematic diagram of CAR molecule constructed with DDR2 nano-antibody 1A12 sequence.
**FIG. 19****:** Ratio of DDR2 positive 293T phagocytosis by macrophages of mice infected with adenovirus packaged with CAR molecules constructed with 1A12.
**FIG. 20****:** Localization image of CAR-M constructed with 1A12.
**FIG. 21****:** Targeted phagocytosis of 293T-DDR2 by CAR-M constructed with 1A12.
**FIG. 22****:** In vivo therapeutic effect of CAR-M constructed with 1A12.
**FIG. 23****:** ⁶⁸Ga PET-CT imaging of BLM mice model by 15 days.

### DETAILED DESCRIPTION

### Definitions

In the present invention, "about" means that a numerical value is within an acceptable error range for the particular value determined by one of ordinary skill in the art, and the numerical value depends in part on how the value is measured or determined (i.e., the limits of the measurement system). For example, "about" may mean a standard deviation within 1 or more than 1 in each practice in the art. Or, "about" or "substantially comprising" may mean a range of up to 20%. Furthermore, particularly for biological systems or processes, the term may mean up to an order of magnitude or up to 5-fold of a numerical value. Unless otherwise specified, when a specific value is provided in the present application and claims, the meaning of "about" or "substantially comprising" should be assumed to be within an acceptable error range for that specific value.

A composition or method described herein as "comprising" one or more named elements or steps is open-ended, meaning that the named elements or steps are essential, but other elements or steps may be added within the scope of the composition or method. It is also understood that any composition or method described as "comprising" one or more named elements or steps also describes the corresponding, more limited composition or method "consisting essentially of" the same named elements or steps, meaning that the composition or method includes the named essential elements or steps and may also include additional elements or steps that do not materially affect the basic and novel characteristic(s) of the composition or method.

As used herein, the term "antibody" refers to any form of antibody that exhibits a desired biological activity (for example inhibiting the binding of a ligand to a receptor thereof, or inhibiting receptor signal transduction induced by a ligand). An "antibody fragment" and an "antigen-binding fragment" refer to an antigen-binding fragment of an antibody and an antibody analogue, which generally include at least a portion of the antigen-binding region or variable region (e.g. one or more CDRs) of a parental antibody. In some embodiments, the antibody is a monoclonal antibody. In some other embodiments, the antibody is a polyclonal antibody.

As used herein, the term "monoclonal antibody" refers to an antibody obtained from a population of substantially homogeneous antibodies, i.e., the individual antibodies that make up the population are identical except for possible natural mutations that may be present in minor amounts. Monoclonal antibodies are highly specific and can be directed against a single antigenic site. Furthermore, in contrast to conventional (polyclonal) antibody preparations, which typically include multiple different antibodies directed against multiple different determinants (epitopes), each monoclonal antibody is directed against only a single determinant on an antigen. The modifier "monoclonal" refers to the properties of an antibody obtained from a substantially homogeneous population of antibodies and should not be construed as requiring any particular method to prepare said antibody. For example, the monoclonal antibodies used for the present invention may be prepared by hybridoma or recombinant DNA.

Monoclonal antibody may include "chimeric" antibody, humanized antibody, or fully humanized antibody. In some embodiments, an antibody consititutes part of a larger biomolecule, such as a fusion protein or an antibody-drug conjugate. An antibody fragment retains at least some of the binding specificity of the parent antibody. Typically, an antibody fragment retains at least 10% of the parental binding activity when that activity is expressed on a molar basis. Preferably, an antibody fragment retains at least 20%, 50%, 70%, 80%, 90%, 95% or 100% or more of the parental antibody's binding affinity for the target.

As used herein, the term "heavy chain antibody" refers to an antibody composed only of heavy chains without light chains, which includes two constant regions (CH2 and CH3), one hinge region, and one heavy chain variable region (i.e. VHH). Examples include but are not limited to natural heavy chain antibodies, natural antibodies without light chains, heavy chain antibodies derived from conventional 4-chain antibodies, and engineered antibodies. Heavy chain antibodies can come from Camelidae species, such as antibodies produced in camels, llamas, dromedaras, alpacas, and pack horses. Other species besides Camelidae can produce heavy chain antibodies that naturally lack light chains; This kind of heavy chain antibody is within the scope of the present invention.

As used in herein, the term "nano-antibody" or "nanobody" refers to a single domain antibody obtained by cloning the variable region of a heavy chain antibody and composed solely of the heavy chain variable region, also known as VHH (variable domain of heavy chain of heavy chain antibody) or single domain antibody. It is the smallest functional antigen binding fragment. Nano-antibodies recognize antigens with high specificity and affinity similar to IgG antibodies, but due to their smaller size (~15kD), they can better penetrate tumor tissues. In addition, nanobodies have resistance to extreme pH, thermal denaturation, protein hydrolysis, solvents, and detergents. They can be expressed and produced with high yield and high solubility.

As used herein, the term "humanized antibody" refers to an antibody that includes CDRs of an antibody derived from a mammal other than human, as well as framework regions (FR) and constant regions of a human antibody.

An "equivalent variant" of an antibody or polypeptide refers to an antibody or polypeptide that has a certain degree of homology or sequence identity with the amino acid sequence of the antibody or polypeptide. In some aspects, the sequence identity is at least about 70%, 75%, 80%, 85%, 90%, 95%, 98%, or 99%. In some aspects, compared to a reference antibody or polypeptide, an equivalent variant thereof has one, two, three, four, or five additions, deletions, substitutions, and combinations thereof. In some aspects, an equivalent variant of an antibody or polypeptide retains the activity (e.g. epitope binding) or structure (e.g. salt bridge) of the reference sequence.

As used herein, a "variant" sequence refers to a sequence that differs from the sequence shown at one or more amino acid residues but retains the biological activity of the resulting molecule.

As used herein, "% identity" between two sequences refers to a function of the number of identical positions shared by the sequences, i.e., % identity = number of equivalent positions/total number of positions x 100, wherein the number of gaps and the length of each gap will be considered, and the gaps need to be introduced when performing an optimal alignment of the two sequences. Sequences alignment and determination of % identity between two sequences may be accomplished using mathematical algorithms.

"Conservative substitution" refers to substitutions of amino acids known to those of skill in this art and may be made generally without altering the biological activity of the resulting molecule. Those of skill in this art recognize that, in general, single amino acid substitutions in non-essential regions of a polypeptide do not alter or substantially alter biological activity. "Do not alter or substantially alter" refers to having a difference of no more than about 20%, about 15%, about 10%, about 9%, about 8%, about 7%, about 6%, about 5%, about 4%, about 3%, about 2%, or about 1% in one or more aspects compared to the object being compared when measured using the same or similar methods.

Generally, CDR3 is considered to play a more important role in antigen recognition than other CDRs. Therefore, in the case of substitution, the present invention prefers conservative substitution of CDRs other than CDR3. In some embodiments, CDR3 is not substituted. Preferred amino acid substitutions include but are not limited to those that: (1) reduce susceptibility to proteolysis, (2) reduce susceptibility to oxidation, (3) alter binding affinity for forming protein complexes, and (4) confer or modify other physicochemical or functional properties of such analogs. Analogs can include various muteins of a sequence other than the naturally-occurring peptide sequence. For example, single or multiple amino acid substitutions (preferably conservative amino acid substitutions) can be made in the naturally-occurring sequence (preferably in the portion of the polypeptide outside the domain(s) forming intermolecular contacts). A conservative amino acid substitution should not substantially change the structural characteristics of the parent sequence (e.g., a replacement amino acid should not tend to break a helix that occurs in the parent sequence, or disrupt other types of secondary structure that characterizes the parent sequence).

It is expected that the binding domain of the antibody or an antigen binding fragment thereof disclosed herein may carry a signal peptide, which is usually located at the N-terminus of the secreted protein and generally consists of 15-30 amino acids. When the signal peptide sequence is synthesized, it is recognized by the signal recognition granule (SRP), protein synthesis is suspended or slowed down, the signal recognition granule carries the ribosome to the endoplasmic reticulum, and the protein synthesis restarts. Under the guidance of the signal peptide, the newly synthesized protein enters the endoplasmic reticulum cavity, and the signal peptide sequence is cleaved under the action of the signal peptidase. If the termination transit sequence exists at the C-terminus of the nascent peptide chain, it may not be cleaved by the signal peptidase, for example, ovalbumin contains an internal signal peptide, and neither its precursor nor the mature form is cleaved by signal peptidase.

According to the present invention, the term "binding" preferably refers to specific binding. When referring to a ligand/receptor, an antibody/antigen or other binding pairs, "specific" binding refers to determining whether there is a binding reaction of the protein in a heterogeneous population of proteins and/or other biochemical reagents. Therefore, under the specified conditions, a specific ligand/antigen binds to a specific receptor/antibody, and does not bind to other proteins present in a sample in a significant amount. "Specifically binds" means that the monoclonal antibody or an antigen binding fragment thereof disclosed herein is capable of specifically interacting with at least two, three, four, five, six, seven, eight or more amino acids of each human target molecule. The "specific binding" of an antibody is mainly characterized by two parameters: a qualitative parameter (binding epitope or antibody binding site) and a quantitative parameter (binding affinity or binding strength). Antibody binding epitopes may be determined by FACS, peptide dot epitope mapping, mass spectrometry, or peptide ELISA. The Biacore and/or ELISA may measure the binding strength of an antibody to a specific epitope. Signal-to-noise ratios are often calculated as a representative measure of binding specificity. In such a signal-to-noise ratio, the signal represents the strength of antibody binding to the target epitope, and the noise represents the strength of antibody binding to other non-target epitopes. Preferably, when the signal-to-noise ratio for a target epitope is about 50, the evaluated antibody may be considered to bind to the target epitope in a specific manner, i.e., "specifically binds". An antigen-binding protein (including an antibody) "specifically binds" to an antigen if it binds to the antigen with high binding affinity as determined by a value of a dissociation constant (KD). In some embodiments, the affinity constant KD is lower than 10⁻⁹ M. As used herein, the term "KD" refers to an equilibrium dissociation constant of a specific antibody-antigen interaction.

As used herein, the term "patient" or "subject" refers to any organism to which a provided antibody, derivative thereof, or composition is or may be administered, for experimental, diagnostic, prophylactic, cosmetic, or therapeutic purposes. Typical p subjects include animals (e.g, mammals such as mice, rats, rabbits, non-human primates, and/or humans). In some embodiments, a subject is a human. In some embodiments, a subject is suffering from or susceptible to one or more disorders or conditions. A patient may display one or more symptoms of a disorder or condition, or may have been diagnosed with one or more disorders or conditions. In some embodiments, a patient is receiving or has received certain therapy to diagnose and/or to treat such disease, disorder, or condition.

The term "treating" or "treatment" as used herein refers to a therapeutic and preventive means in which the occurrence or progression of an undesired pathological change or disorder is prevented from or slowed in a subject. Beneficial or desirable clinical outcomes include, but are not limited to, alleviation of symptoms, reduction in disease severity, stabilization of disease states (i.e., no deterioration), delay or slowing of disease progression, reduction or mitigation of disease states, and local or holistic cure of disease, whether or not these results are detectable. "Treatment" can also refer to prolonged survival as compared to the expected survival without treatment. Subjects in need of treatment include those already having the disease or condition as well as those who are prone to have the disease or condition or who are in need of prevention from the disease or condition.

The term "prevention" as used herein includes the prevention or mitigation of the onset of clinically significant disease progression or the onset of preclinically significant disease stages in individuals at risk. This includes the preventive treatment of individuals at risk of disease development.

When "administering" and "treating" or "preventing" refer to an animal, human, experimental subject, cell, tissue, organ or biological fluid, it refers to contacting the animal, human, subject, cell, tissue, organ or biological fluid with an exogenous drug, therapeutic agent, diagnostic agent or composition. "Administration" and "treatment" may refer to, for example, therapeutic methods, pharmacokinetic methods, diagnostic methods, research methods, and experimental methods. Treatment of the cells includes contacting the agent with the cells and contacting the agent with a fluid, wherein the fluid is in contact with the cells. "Administration" and "treatment" also mean *in vitro* and ex *vivo* treatment of cells, e.g., by agents, diagnostic agents, binding compositions, or by other cells.

As used herein, the term "therapeutically effective amount" or "effective amount" refers to when the antibody or derivative thereof disclosed herein is administered alone or in combination with another therapeutic agent to a cell, tissue or subject, it effectively prevents or slows the amount of the disease or condition to be treated. A therapeutically effective dose further refers to the amount sufficient to cause alleviation of symptoms, such as treating, curing, preventing or alleviating related medical conditions, or improving the treatment rate, cure rate, prevention rate, or alleviation rate of the symptoms. When an active ingredient alone is administered to an individual, the therapeutically effective amount refers to the mount of the alone ingredient. When a combination is administered, the therapeutically effective amount refers to the combined amount of active ingredients that produce a therapeutic effect, regardless of whether it is administered in combination, sequentially or simultaneously. A therapeutically effective amount will reduce symptoms usually by at least 10%; usually at least 20%; preferably at least about 30%; more preferably at least 40% and most preferably at least 50%.

As used herein, "pharmaceutically acceptable carrier" includes a material which, when combined with an active ingredient of a composition, allows the ingredient to retain biological activity and without causing disruptive reactions with the subject's immune system. Such may include stabilizers, preservatives, salt or sugar complexes or crystals, and the like. "Pharmaceutically acceptable" refers to molecules and ingredients that will not produce allergic reactions or similar undesirable reactions when applied to human body.

As used herein, a "chimeric antigen receptor (CAR)" means a fused protein comprising an extracellular domain capable of binding to an antigen, a transmembrane domain derived from a polypeptide different from a polypeptide from which the extracellular domain is derived, and at least one intracellular domain. The "chimeric antigen receptor (CAR)" is sometimes called a "chimeric receptor", a "T-body", or a "chimeric immune receptor (CIR)." The "extracellular domain capable of binding to an antigen" means any oligopeptide or polypeptide that can bind to a certain antigen. The "intracellular domain" means any oligopeptide or polypeptide known to function as a domain that transmits a signal to cause activation or inhibition of a biological process in a cell.

The term "antibody-drug conjugate" or "ADC" refers to an antibody that has covalently conjugated to a therapeutic active substance or active pharmaceutical ingredient, so that the therapeutic active substance or active pharmaceutical ingredient can target the binding target of the antibody to demonstrate its pharmacological function. The therapeutically active substance or active pharmaceutical ingredient may be a cytotoxin capable of killing cells targeted by ADC. The covalent bonding of therapeutic active substances, active pharmaceutical ingredients, or cytotoxics can be performed in a non-site specific manner using standard chemical linkers that couple payloads to lysine or cysteine residues, or preferably, the conjugation is performed in a site-specific manner, which allows full control of the conjugation site and the drug/antibody ratio (DAR) to the generated ADC.

The term "siRNA" refers to a double-stranded RNA with a length of 20 to 25 nucleotides, comprising two anti-parallel and substantially complementary nucleic acid strands, referred to as having "sense" and "antisense" orientations with respect to a target RNA. A siRNA moiety triggers the degradation of a target RNA, e.g., an mRNA, through a post-transcriptional gene-silencing mechanism referred to herein as RNA interference or RNAi. In general, the majority of nucleotides of each strand of a siRNA moiety are ribonucleotides, but each or both strands can also include one or more non-ribonucleotides, e.g., a deoxyribonucleotide and/or a modified nucleotide. In addition, a siRNA moiety may include ribonucleotides with chemical modifications, and may also include substantial modifications at multiple nucleotides. Such modifications may include all types of modifications disclosed herein or known in the art. Any such modifications are intended to be encompassed within the range of the definition of "siRNA".

The term "survival genes" can be used interchangeably with "essential genes", which refers to the genes necessary for the survival or reproduction of a specific cell. The functional defect of one of these genes will cause the cell to have a lethal phenotype and cannot survive or reproduce. Typical survival genes are those encoding key proteases in the process of transcription, replication and expression of genetic material, including but not limited to POLR2A (DNA-directed RNA polymerase II subunit RPB1, the largest subunit of RNA polymerase II, which can synthesize mRNA precursors and many functional non coding RNAs, and form the polymerase active center with the second largest subunit), POLR2B (DNA-directed RNA polymerase II subunit RPB2, the second largest subunit of RNA polymerase II, forms the polymerase active center together with the largest subunit), DKC1 (H/ACA ribonucleoprotein complex subunit DKC1), CENPE (Centromere-associated protein E), elF-3b (eukaryotic translation initiation factor 3 subunit b), etc. Accordingly, the term "a survival gene in a DDR2 positive cell" refers to a gene necessary for the survival or reproduction of DDR2 positive cells. The method of determining whether a specific gene in human cells is an essential gene is known in the art. For example, see Wang T et al. Identification and characterization of essential genes in the human genome. Science 27 Nov 2015: Vol. 350, Issue 6264, pp. 1096-1101 or Vincent A. Blomen et al., Gene essentiality and synthetic lethality in haploid human cells. Science 27 Nov 2015: Vol. 350, Issue 6264, pp. 1092-1096.

### Anti-DDR2 Nano-antibody

The inventors immunized alpacas with human DDR2 extracellular segment (the amino acid sequence is UniProtKB/Swiss-Prot: Q16832.2 aa 22 to aa 399) to obtain camel derived heavy chain antibodies, and then obtained corresponding nano-antibodies.

In an aspect, the present invention provides a nano-antibody binding to DDR2, wherein the nano-antibody comprises a CDR1, a CDR2, and a CDR3 comprising or being the CDR1, CDR2, and CDR3 of a nano-antibody selected from the amino acid sequence of any one of SEQ ID NO: 4, SEQ ID NO: 8, SEQ ID NO: 12, SEQ ID NO: 16, and SEQ ID NO: 20, or an equivalent variant of each thereof.

In some embodiments, the CDR1, CDR1, and CDR3 are defined based on any one of the IMGT, Kabat, Chothia, Contact, or Martin definition scheme. In some embodiments, the CDR1, CDR1, and CDR3 are defined based on the IMGT definition scheme.

In some embodiments, the nano-antibody comprises a CDR1, a CDR2, and a CDR3, and:
(a) the CDR1 comprises or is the sequence set forth in SEQ ID NO: 1 or an equivalent variant thereof, the CDR2 comprises or is the sequence set forth in SEQ ID NO: 2 or an equivalent variant thereof, the CDR3 comprises or is the sequence set forth in SEQ ID NO: 3 or an equivalent variant thereof;
(b) the CDR1 comprises or is the sequence set forth in SEQ ID NO: 5 or an equivalent variant thereof, the CDR2 comprises or is the sequence set forth in SEQ ID NO: 6 or an equivalent variant thereof, the CDR3 comprises or is the sequence set forth in SEQ ID NO: 7 or an equivalent variant thereof;
(c) the CDR1 comprises or is the sequence set forth in SEQ ID NO: 9 or an equivalent variant thereof, the CDR2 comprises or is the sequence set forth in SEQ ID NO: 10 or an equivalent variant thereof, the CDR3 comprises or is the sequence set forth in SEQ ID NO: 11 or an equivalent variant thereof;
(d) the CDR1 comprises or is the sequence set forth in SEQ ID NO: 13 or an equivalent variant thereof, the CDR2 comprises or is the sequence set forth in SEQ ID NO: 14 or an equivalent variant thereof, the CDR3 comprises or is the sequence set forth in SEQ ID NO: 15 or an equivalent variant thereof; or
(e) the CDR1 comprises or is the sequence set forth in SEQ ID NO: 17 or an equivalent variant thereof, the CDR2 comprises or is the sequence set forth in SEQ ID NO: 18 or an equivalent variant thereof, the CDR3 comprises or is the sequence set forth in SEQ ID NO: 19 or an equivalent variant thereof.

In some embodiments, the nano-antibody comprises or is the sequence selected from any one of SEQ ID NO: 4, SEQ ID NO: 8, SEQ ID NO: 12, SEQ ID NO: 16, and SEQ ID NO: 20, and an equivalent variant of each thereof.

In some embodiments, the equivalent variant of each of the CDR1, CDR2 and CDR3 refers to one that has a single amino acid substitution, deletion or insertion compared to the reference sequence.

In some embodiments, the equivalent variant of each of the nano-antibodies refers to one that has at least 75%, 80%, 85%, 90%, 95%, 98%, or 99% sequence identity with the sequence of any one of SEQ ID NO: 4, SEQ ID NO: 8, SEQ ID NO: 12, SEQ ID NO: 16, and SEQ ID NO: 20, and has the same or equivalent CDR1, CDR2 and CDR3.

In some embodiments, the nano-antibody comprises a CDR1, a CDR2, and a CDR3, and:
(a) the CDR1 comprises the sequence set forth in SEQ ID NO: 1, the CDR2 comprises the sequence set forth in SEQ ID NO: 2, the CDR3 comprises the sequence set forth in SEQ ID NO: 3;
(b) the CDR1 comprises the sequence set forth in SEQ ID NO: 5, the CDR2 comprises the sequence set forth in SEQ ID NO: 6, the CDR3 comprises the sequence set forth in SEQ ID NO: 7;
(c) the CDR1 comprises the sequence set forth in SEQ ID NO: 9, the CDR2 comprises the sequence set forth in SEQ ID NO: 10, the CDR3 comprises the sequence set forth in SEQ ID NO: 11;
(d) the CDR1 comprises the sequence set forth in SEQ ID NO: 13, the CDR2 comprises the sequence set forth in SEQ ID NO: 14, the CDR3 comprises the sequence set forth in SEQ ID NO: 15; or
(e) the CDR1 comprises the sequence set forth in SEQ ID NO: 17, the CDR2 comprises the sequence set forth in SEQ ID NO: 18, the CDR3 comprises the sequence set forth in SEQ ID NO: 19.

In some embodiments, the nano-antibody comprises the sequence selected from any one of SEQ ID NO: 4, SEQ ID NO: 8, SEQ ID NO: 12, SEQ ID NO: 16, and SEQ ID NO: 20.

In some embodiments, the nano-antibody comprises a CDR1, a CDR2, and a CDR3, and:
(a) the CDR1 is the sequence set forth in SEQ ID NO: 1, the CDR2 is the sequence set forth in SEQ ID NO: 2, the CDR3 is the sequence set forth in SEQ ID NO: 3;
(b) the CDR1 is the sequence set forth in SEQ ID NO: 5, the CDR2 is the sequence set forth in SEQ ID NO: 6, the CDR3 is the sequence set forth in SEQ ID NO: 7;
(c) the CDR1 is the sequence set forth in SEQ ID NO: 9, the CDR2 is the sequence set forth in SEQ ID NO: 10, the CDR3 is the sequence set forth in SEQ ID NO: 11;
(d) the CDR1 is the sequence set forth in SEQ ID NO: 13, the CDR2 is the sequence set forth in SEQ ID NO: 14, the CDR3 is the sequence set forth in SEQ ID NO: 15; or
(e) the CDR1 is the sequence set forth in SEQ ID NO: 17, the CDR2 is the sequence set forth in SEQ ID NO: 18, the CDR3 is the sequence set forth in SEQ ID NO: 19.

In some embodiments, the nano-antibody is the sequence selected from any one of SEQ ID NO: 4, SEQ ID NO: 8, SEQ ID NO: 12, SEQ ID NO: 16, and SEQ ID NO: 20.

In some embodiments, the substitutions disclosed herein are conservative substitutions.

A "conservative amino acid substitution" is one in which the amino acid residue is replaced with an amino acid residue having a similar side chain. Families of amino acid residues having similar side chains have been defined in the art, including basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine). Thus, a nonessential amino acid residue in an immunoglobulin polypeptide is preferably replaced with another amino acid residue from the same side chain family. In another embodiment, a string of amino acids can be replaced with a structurally similar string that differs in order and/or composition of side chain family members.

It will also be understood by one of skill in the art that antibodies as disclosed herein may be modified such that they vary in amino acid sequence from the naturally occurring binding polypeptide from which they were derived. For example, a polypeptide or amino acid sequence derived from a designated protein may be similar, e.g., have a certain percent identity to the starting sequence, e.g., it may be 60%, 70%, 75%, 80%, 85%, 90%, 95%, 98%, 99%, or a range between any two of these values, identical to the starting sequence.

In some embodiments, the antibody comprises an amino acid sequence or one or more moieties not normally associated with an antibody. Exemplary modifications are described in more detail herein. For example, an antibody disclosed herein may comprise a flexible linker sequence, or may be modified to add a functional moiety (e.g., polyethylene glycol (PEG), a drug, a toxin, or a label).

Antibodies, variants, or derivatives thereof of the disclosure include derivatives that are modified, i.e., by the covalent attachment of any type of molecule to the antibody such that covalent attachment does not prevent the antibody from binding to the epitope. For example, but not by way of limitation, the antibodies can be modified, e.g., by glycosylation, acetylation, pegylation, phosphorylation, phosphorylation, amidation, derivatization by known protecting/blocking groups, proteolytic cleavage, linkage to a cellular ligand or other protein, etc. Any of numerous chemical modifications may be carried out by known techniques, including, but not limited to specific chemical cleavage, acetylation, formylation, metabolic synthesis of tunicamycin, etc. Additionally, the antibodies may contain one or more non-classical amino acids.

In some embodiments, the antibodies may be conjugated to therapeutic agents, prodrugs, peptides, proteins, enzymes, viruses, lipids, biological response modifiers, pharmaceutical agents, or PEG.

The antibodies may be conjugated or fused to a therapeutic agent, which may include detectable labels such as radioactive labels, an immunomodulator, a hormone, an enzyme, an oligonucleotide, a photoactive therapeutic or diagnostic agent, a cytotoxic agent, which may be a drug or a toxin, an ultrasound enhancing agent, a non-radioactive label, a combination thereof and other such agents known in the art.

The antibodies can be detectably labeled by coupling it to a chemiluminescent compound. The presence of the chemiluminescent-tagged antigen-binding polypeptide is then determined by detecting the presence of luminescence that arises during the course of a chemical reaction. Examples of particularly useful chemiluminescent labeling compounds are luminol, isoluminol, theromatic acridinium ester, imidazole, acridinium salt and oxalate ester.

The antibodies can also be detectably labeled using fluorescence emitting metals such as ¹⁵²Eu, or others of the lanthanide series. These metals can be attached to the antibody using such metal chelating groups as diethylenetriaminepentacetic acid (DTPA) or ethylenediaminetetraacetic acid (EDTA). Techniques for conjugating various moieties to an antibody are well known.

### Humanized Antibody

In any aspect of the present invention, the nano-antibody binding to DDR2 is preferably a humanized antibody.

Humanized antibodies are antibody molecules derived from a non-human species antibody that bind the desired antigen having one or more complementarity determining regions (CDRs) from the non-human species and framework regions from a human immunoglobulin molecule. Often, framework residues in the human framework regions will be substituted with the corresponding residue from the CDR donor antibody to alter, preferably improve, antigen-binding. These framework substitutions are identified by methods well known in the art, e.g., by modeling of the interactions of the CDR and framework residues to identify framework residues important for antigen-binding and sequence comparison to identify unusual framework residues at particular positions. Antibodies can be humanized using a variety of techniques known in the art including, for example, CDR-grafting, veneering or resurfacing, and chain shuffling, ect.

Completely human antibodies are particularly desirable for therapeutic treatment of human patients. Human antibodies can be made by a variety of methods known in the art including phage display methods using antibody libraries derived from human immunoglobulin sequences.

Human antibodies can also be produced using transgenic mice which are incapable of expressing functional endogenous immunoglobulins, but which can express human immunoglobulin genes. For example, the human heavy and light chain immunoglobulin gene complexes may be introduced randomly or by homologous recombination into mouse embryonic stem cells. Alternatively, the human variable region, constant region, and diversity region may be introduced into mouse embryonic stem cells in addition to the human heavy and light chain genes. The mouse heavy and light chain immunoglobulin genes may be rendered non-functional separately or simultaneously with the introduction of human immunoglobulin loci by homologous recombination. In particular, homozygous deletion of the JH region prevents endogenous antibody production. The modified embryonic stem cells are expanded and microinjected into blastocysts to produce chimeric mice. The chimeric mice are then bred to produce homozygous offspring that express human antibodies. The transgenic mice are immunized in the normal fashion with a selected antigen, e.g., all or a portion of a desired target polypeptide. Monoclonal antibodies directed against the antigen can be obtained from the immunized, transgenic mice using conventional hybridoma technology. The human immunoglobulin transgenes harbored by the transgenic mice rearrange during B-cell differentiation, and subsequently undergo class switching and somatic mutation. Thus, using such a technique, it is possible to produce therapeutically useful IgG, IgA, IgM and IgE antibodies.

Completely human antibodies which recognize a selected epitope can also be generated using a technique referred to as "guided selection". In this approach a selected non-human monoclonal antibody, e.g., a mouse antibody, is used to guide the selection of a completely human antibody recognizing the same epitope.

DNA encoding desired monoclonal antibodies can be readily isolated and sequenced using conventional procedures (e.g., by using oligonucleotide probes that are capable of binding specifically to genes encoding the heavy and light chains of murine antibodies). The isolated and subcloned hybridoma cells serve as a preferred source of such DNA. Once isolated, the DNA may be placed into expression vectors, which are then transfected into prokaryotic or eukaryotic host cells such as E. coli cells, simian COS cells, Chinese Hamster Ovary (CHO) cells or myeloma cells that do not otherwise produce immunoglobulins. More particularly, the isolated DNA may be used to clone constant and variable region sequences for the manufacture of antibodies. Essentially, this entails extraction of RNA from the selected cells, conversion to cDNA, and amplification by PCR using Ig specific primers. As described herein, transformed cells expressing the desired antibody can be grown up in relatively large quantities to provide clinical and commercial supplies of the immunoglobulin.

Additionally, using routine recombinant DNAtechniques, one or more of the CDRs of the antigen-binding polypeptides of the present disclosure, may be inserted within framework regions, e.g., into human framework regions to humanize a non-human antibody. The framework regions may be naturally occurring or consensus framework regions, and preferably human framework regions. Preferably, the polynucleotide generated by the combination of the framework regions and CDRs encodes an antibody that specifically binds to at least one epitope of a desired polypeptide, e.g., LIGHT. Preferably, one or more amino acid substitutions may be made within the framework regions, and, preferably, the amino acid substitutions improve binding of the antibody to its antigen. Additionally, such methods may be used to make amino acid substitutions or deletions of one or more variable region cysteine residues participating in an intrachain disulfide bond to generate antibody molecules lacking one or more intrachain disulfide bonds. Other alterations to the polynucleotide are encompassed by the present disclosure and within the skill of the art.

For Camelidae antibodies, especially heavy chain antibodies and nano-antibodies (VHH), humanising a polypeptide, according to the present invention, comprises a step of replacing one or more of the Camelidae amino acids by their human counterpart as found in the human consensus sequence, without that polypeptide losing its typical character, i.e. the humanisation does not significantly affect the antigen binding capacity of the resulting polypeptide. Such methods are known by the skilled addressee.

In an embodiment, the "humanization" of VHH refers to replacing one or more amino acid residues in the amino acid sequence of the naturally occurring VHH sequence (and in particular in the framework sequences) by one or more of the amino acid residues that occur at the corresponding position(s) in a VH domain from a conventional 4-chain antibody from a human being. This can be performed using humanization techniques known in the art. In some embodiments, possible humanizing substitutions or combinations of humanizing substitutions may be determined by methods known in the art, for example, by a comparison between the sequence of a VHH and the sequence of a naturally occurring human VH domain. In some embodiments, the humanizing substitutions are chosen such that the resulting humanized VHHs still retain advantageous functional properties. Generally, as a result of humanization, the VHHs of the present application may become more "human-like", while still retaining favorable properties such as a reduced immunogenicity, compared to the corresponding naturally occurring VHH domains. In various embodiments, the humanized VHHs of the present application can be obtained in any suitable manner known in the art and thus are not strictly limited to polypeptides that have been obtained using a polypeptide that comprises a naturally occurring VHH domain as a starting material.

In various embodiments, both "humanization" and "camelization" can be performed by providing a nucleotide sequence that encodes a naturally occurring VHH domain or VH domain, respectively, and then changing, in a manner known in the art, one or more codons in the nucleotide sequence in such a way that the new nucleotide sequence encodes a "humanized" or "camelized" VHH, respectively. This nucleic acid can then be expressed in a manner known in the art, so as to provide the desired VHH of the present application. Alternatively, based on the amino acid sequence of a naturally occurring VHH domain or VH domain, respectively, the amino acid sequence of the desired humanized or camelized VHH of the present application, respectively, can be designed and then synthesized de novo using techniques for peptide synthesis known in the art. Also, based on the amino acid sequence or nucleotide sequence of a naturally occurring VHH domain or VH domain, respectively, a nucleotide sequence encoding the desired humanized or camelized VHH, respectively, can be designed and then synthesized de novo using techniques for nucleic acid synthesis known in the art, after which the nucleic acid thus obtained can be expressed in a manner known in the art, so as to provide the desired VHH of the present application. Other suitable methods and techniques for obtaining the VHHs of the invention and/or nucleic acids encoding the same, starting from naturally occurring VH sequences or VHH sequences, are known in the art, and may, for example, comprise combining one or more parts of one or more naturally occurring VH sequences (such as one or more FR sequences and/or CDR sequences), one or more parts of one or more naturally occurring VHH sequences (such as one or more FR sequences or CDR sequences), and/or one or more synthetic or semisynthetic sequences, in a suitable manner, so as to provide a VHH of the present application or a nucleotide sequence or nucleic acid encoding the same.

Accordingly, the present invention provides a humanized nano-antibody binding to DDR2, wherein the nano-antibody comprises a CDR1, a CDR2, and a CDR3, and:
(a) the CDR1 comprises or is the sequence set forth in SEQ ID NO: 1 or an equivalent variant thereof, the CDR2 comprises or is the sequence set forth in SEQ ID NO: 2 or an equivalent variant thereof, the CDR3 comprises or is the sequence set forth in SEQ ID NO: 3 or an equivalent variant thereof;
(b) the CDR1 comprises or is the sequence set forth in SEQ ID NO: 5 or an equivalent variant thereof, the CDR2 comprises or is the sequence set forth in SEQ ID NO: 6 or an equivalent variant thereof, the CDR3 comprises or is the sequence set forth in SEQ ID NO: 7 or an equivalent variant thereof;
(c) the CDR1 comprises or is the sequence set forth in SEQ ID NO: 9 or an equivalent variant thereof, the CDR2 comprises or is the sequence set forth in SEQ ID NO: 10 or an equivalent variant thereof, the CDR3 comprises or is the sequence set forth in SEQ ID NO: 11 or an equivalent variant thereof;
(d) the CDR1 comprises or is the sequence set forth in SEQ ID NO: 13 or an equivalent variant thereof, the CDR2 comprises or is the sequence set forth in SEQ ID NO: 14 or an equivalent variant thereof, the CDR3 comprises or is the sequence set forth in SEQ ID NO: 15 or an equivalent variant thereof; or
(e) the CDR1 comprises or is the sequence set forth in SEQ ID NO: 17 or an equivalent variant thereof, the CDR2 comprises or is the sequence set forth in SEQ ID NO: 18 or an equivalent variant thereof, the CDR3 comprises or is the sequence set forth in SEQ ID NO: 19 or an equivalent variant thereof.

In another aspect, the present invention provides a polynucleotide comprising or being a polynucleotide encoding the antibody according to any of the aspects described above. In some embodiments, the polynucleotide comprises or is the sequence selected from any one of SEQ ID NO: 21, SEQ ID NO: 22, SEQ ID NO: 23, SEQ ID NO: 24, and SEQ ID NO: 25, and an equivalent variant of each thereof.

### Chimeric Antigen Receptor (CAR)

Chimeric antigen receptor (CAR) T cells are those T cells on whose surface chimeric receptors which can recognize specific antigens and can transmit signals are expressed. CAR T cells play an important role in treating tumors by expressing chimeric antigen receptor (CAR) molecules which typically include an extracellular segment, a transmembrane region and an intracellular segment: the extracellular segment is a single-chain variable fragment (ScFv) formed by connecting heavy chain and light chain variable regions of an antibody via a peptide fragment; the intracellular segment is intracellular chimeras consisting of a variety of signaling molecules; and the transmembrane region is originated from the transmembrane region of other molecules. The single chain variable fragment gene is isolated from, for example, hybridomas capable of generating monoclonal antibody which recognizes a target antigen. T cells expressing CAR molecules directly recognize antigens on the surface of tumor cells independent of the expression of major histocompatibility antigen type I on tumor cells, and at the same time activate T cells. Therefore, the T cells expressing CARs can kill tumor cells effectively. Briefly, CAR T cells recognize specific molecules on the surface of tumor cells through antigen-antibody recognition manner, and then experience activation and proliferation and exert cytotoxic function through their intracellular signaling.

Accordingly, in another aspect, the present invention provides a chimeric antigen receptor, comprising an extracellular domain capable of binding to an antigen, a transmembrane domain, and an intracellular domain, wherein the extracellular domain capable of binding to an antigen comprises any one of the nano-antibodies described above.

In some embodiments, the CAR of the present invention may comprise, for example, the following transmembrane domains: T cell acceptor alpha or beta chain, CD3 zeta chain, CD28, CD3ε, CD45, CD4, CD5, CD8, CD9, CD16, CD22, CD33, CD37, CD64, CD80, CD86, CD134, CD137, ICOS, CD154, Tlr4, FcγR I, FcγR II, FcγR III, FCER1, or CD36. In some embodiments, the transmembrane domain comprises CD8α or CD28. In some embodiments, the transmembrane domain comprises a Tlr4 transmembrane region.

In some embodiments, the CAR of the present invention may comprise, for example, the following intracellular signaling domains: CD3ζ, CD28, 4-1BB (or CD137), CD27, and OX40 (or CD134). In some embodiments, the intracellular domain comprises CD3 zeta and one of CD28, 4-1BB (or CD137), CD27 and OX40 (or CD134). In some embodiments, the intracellular domain comprises CD3 zeta and two of CD28, 4-1BB (or CD137), CD27 and OX40 (or CD134). In some embodiments, the intracellular domain comprises one or more of the CD86 intracellular functional region, the Tlr4 CSD region (that is, the Tlr4 intracellular Toll-Il-1 receptor1 (TIR) domain), and the FcγR I intracellular functional region. In some embodiments, the intracellular domain comprises a Tlr4 CSD region and an FcγR I intracellular functional region.

Accordingly, the present invention provides a chimeric antigen receptor, comprising an extracellular domain capable of binding to an antigen, a transmembrane domain, and an intracellular domain, wherein the extracellular domain capable of binding to an antigen comprises any one of the nano-antibodies described above, the transmembrane domain comprises a Tlr4 transmembrane region, and the intracellular domain comprises a Tlr4 CSD region and an FcγR I intracellular functional region.

Accordingly, the present invention further provides a modified immune cell expressing any one of the chimeric antigen receptors described above.

In some embodiments, the immune cell is selected from T cell, NK-cell, macrophage, and neutrophil. In some embodiments, the T cells is a cytotoxic T lymphocyte, NKT cell, helper T cell, or suppressor/and regulatory T cell. In some embodiments, the macrophage is a type M1 macrophage or a type M2 macrophage.

In some embodiments, the present invention provides a CAR-T cell expressing a chimeric antigen acceptor, wherein the extracellular domain of the chimeric antigen acceptor comprises any one of the nano-antibodies described herein.

In some embodiments, the present invention provides a CAR-T cell expressing a chimeric antigen acceptor, wherein the extracellular domain of the chimeric antigen acceptor comprises any one of the nano-antibodies described herein, wherein the nano-antibody comprises a CDR1, a CDR2, and a CDR3, and:
(a) the CDR1 comprises or is the sequence set forth in SEQ ID NO: 1 or an equivalent variant thereof, the CDR2 comprises or is the sequence set forth in SEQ ID NO: 2 or an equivalent variant thereof, the CDR3 comprises or is the sequence set forth in SEQ ID NO: 3 or an equivalent variant thereof;
(b) the CDR1 comprises or is the sequence set forth in SEQ ID NO: 5 or an equivalent variant thereof, the CDR2 comprises or is the sequence set forth in SEQ ID NO: 6 or an equivalent variant thereof, the CDR3 comprises or is the sequence set forth in SEQ ID NO: 7 or an equivalent variant thereof;
(c) the CDR1 comprises or is the sequence set forth in SEQ ID NO: 9 or an equivalent variant thereof, the CDR2 comprises or is the sequence set forth in SEQ ID NO: 10 or an equivalent variant thereof, the CDR3 comprises or is the sequence set forth in SEQ ID NO: 11 or an equivalent variant thereof;
(d) the CDR1 comprises or is the sequence set forth in SEQ ID NO: 13 or an equivalent variant thereof, the CDR2 comprises or is the sequence set forth in SEQ ID NO: 14 or an equivalent variant thereof, the CDR3 comprises or is the sequence set forth in SEQ ID NO: 15 or an equivalent variant thereof; or
(e) the CDR1 comprises or is the sequence set forth in SEQ ID NO: 17 or an equivalent variant thereof, the CDR2 comprises or is the sequence set forth in SEQ ID NO: 18 or an equivalent variant thereof, the CDR3 comprises or is the sequence set forth in SEQ ID NO: 19 or an equivalent variant thereof.

In some embodiments, the present invention provides a CAR-M cell (macrophage) expressing a chimeric antigen acceptor, wherein the extracellular domain of the chimeric antigen acceptor comprises any one of the nano-antibodies described herein.

In some embodiments, the present invention provides a CAR-M cell expressing a chimeric antigen acceptor, wherein the extracellular domain of the chimeric antigen acceptor comprises any one of the nano-antibodies described herein, wherein the nano-antibody comprises a CDR1, a CDR2, and a CDR3, and:
(a) the CDR1 comprises or is the sequence set forth in SEQ ID NO: 1 or an equivalent variant thereof, the CDR2 comprises or is the sequence set forth in SEQ ID NO: 2 or an equivalent variant thereof, the CDR3 comprises or is the sequence set forth in SEQ ID NO: 3 or an equivalent variant thereof;
(b) the CDR1 comprises or is the sequence set forth in SEQ ID NO: 5 or an equivalent variant thereof, the CDR2 comprises or is the sequence set forth in SEQ ID NO: 6 or an equivalent variant thereof, the CDR3 comprises or is the sequence set forth in SEQ ID NO: 7 or an equivalent variant thereof;
(c) the CDR1 comprises or is the sequence set forth in SEQ ID NO: 9 or an equivalent variant thereof, the CDR2 comprises or is the sequence set forth in SEQ ID NO: 10 or an equivalent variant thereof, the CDR3 comprises or is the sequence set forth in SEQ ID NO: 11 or an equivalent variant thereof;
(d) the CDR1 comprises or is the sequence set forth in SEQ ID NO: 13 or an equivalent variant thereof, the CDR2 comprises or is the sequence set forth in SEQ ID NO: 14 or an equivalent variant thereof, the CDR3 comprises or is the sequence set forth in SEQ ID NO: 15 or an equivalent variant thereof; or
(e) the CDR1 comprises or is the sequence set forth in SEQ ID NO: 17 or an equivalent variant thereof, the CDR2 comprises or is the sequence set forth in SEQ ID NO: 18 or an equivalent variant thereof, the CDR3 comprises or is the sequence set forth in SEQ ID NO: 19 or an equivalent variant thereof.

In some embodiments, the nano-antibody comprises or is the sequence selected from any one of SEQ ID NO: 4, SEQ ID NO: 8, SEQ ID NO: 12, SEQ ID NO: 16, and SEQ ID NO: 20 and an equivalent variant of each thereof.

### Bispecific Antibody

A bispecific antibody (BsAb) is an artificial antibody that can specifically recognize and bind to two different antigens or epitopes. If the two antigens are located on surface of different cells, the bispecific antibody can set up a bridge between the two antigen molecules, thereby forming cross-links between cells and mediating the cells to produce directed effector functions. Bispecific antibodies used for immunotherapy are artificial antibodies containing two specific antigen binding sites that bind to cell receptor antigens, and they can set up a bridge between diseased cells (target cells) and functional cells (immune cells), to stimulate a directed immune response. The killing of tumor cells by BsAb-mediated immune cells (such as T cells, NK cells and the like) is currently a hot spot in the application research of immunotherapy. The mechanism of action is that BsAb can simultaneously bind to tumor-related antigens and target molecules on immune effector cells, and directly leads to the specific killing of tumor cells by immune effector cells while activating immune cells.

Accordingly, in another aspect, the present invention provides a bispecific antibody, comprising a first binding moiety binding to a first antigen and a second binding moiety binding to a second antigen, wherein the first binding moiety comprises any one of the nano-antibodies described above.

In some embodiments, the second antigen targeted by the bispecific antibody is a specific antigen on the surface of an immune cell. In some embodiments, the second antigen is an antigen or epitope of a B cell, a T cell, a myeloid cell, a plasma cell, or a mast cell.

In some embodiments, the second antigen is selected from CD3, CD4, CD8, CD20, CD19, CD21, CD23, CD46, CD80, HLA-DR, CD74, CD22, CD14, CD15, CD16, CD89, CD123, TCRγ/δ, NKp46, and KIR.

In some embodiments, the second antigen is CD16a. CD16a (FcγRIIIa) is a low affinity acceptor of the IgG Fc domain, which is involved in antibody dependent cytotoxicity (ADCC) and is responsible for triggering cell lysis of target cells through natural killing (NK).

In some embodiments, the second antigen is CD89. CD89, also known as the immunoglobulin α Fc receptor (Fc α RI), is a glycoprotein that is expressed on the surface of neutrophils, monocytes, macrophages and eosinophils, and is a potent cytotoxic trigger.

In some embodiments, the second antigen is CD3. CD3 exists only on the surface of T cells and is composed of six peptide chains. It often binds closely with T cell receptor (TCR) to form a TCR-CD3 complex containing eight peptide chains, which is jointly involved in antigen recognition and signal transduction by T cells.

In some embodiments, the present invention provides a bispecific antibody, comprising a first binding moiety binding to a first antigen and a second binding moiety binding to a second antigen, wherein the first antigen is DDR2 and the first binding moiety comprises a nano-antibody, and the second antigen is an antigen or epitope of a B cell, a T cell, a myeloid cell, a plasma cell, or a mast cell, preferably a T cell surface antigen, preferably CD3, wherein the nano-antibody comprises a CDR1, a CDR2, and a CDR3, and:
(a) the CDR1 comprises or is the sequence set forth in SEQ ID NO: 1 or an equivalent variant thereof, the CDR2 comprises or is the sequence set forth in SEQ ID NO: 2 or an equivalent variant thereof, the CDR3 comprises or is the sequence set forth in SEQ ID NO: 3 or an equivalent variant thereof;
(b) the CDR1 comprises or is the sequence set forth in SEQ ID NO: 5 or an equivalent variant thereof, the CDR2 comprises or is the sequence set forth in SEQ ID NO: 6 or an equivalent variant thereof, the CDR3 comprises or is the sequence set forth in SEQ ID NO: 7 or an equivalent variant thereof;
(c) the CDR1 comprises or is the sequence set forth in SEQ ID NO: 9 or an equivalent variant thereof, the CDR2 comprises or is the sequence set forth in SEQ ID NO: 10 or an equivalent variant thereof, the CDR3 comprises or is the sequence set forth in SEQ ID NO: 11 or an equivalent variant thereof;
(d) the CDR1 comprises or is the sequence set forth in SEQ ID NO: 13 or an equivalent variant thereof, the CDR2 comprises or is the sequence set forth in SEQ ID NO: 14 or an equivalent variant thereof, the CDR3 comprises or is the sequence set forth in SEQ ID NO: 15 or an equivalent variant thereof; or
(e) the CDR1 comprises or is the sequence set forth in SEQ ID NO: 17 or an equivalent variant thereof, the CDR2 comprises or is the sequence set forth in SEQ ID NO: 18 or an equivalent variant thereof, the CDR3 comprises or is the sequence set forth in SEQ ID NO: 19 or an equivalent variant thereof.

In some embodiments, the present invention provides a bispecific antibody, comprising a first binding moiety binding to a first antigen and a second binding moiety binding to a second antigen, wherein the first antigen is DDR2 and the first binding moiety comprises a nano-antibody, the nano-antibody comprises a CDR1, a CDR2, and a CDR3, and:
(a) the CDR1 comprises or is the sequence set forth in SEQ ID NO: 1 or an equivalent variant thereof, the CDR2 comprises or is the sequence set forth in SEQ ID NO: 2 or an equivalent variant thereof, the CDR3 comprises or is the sequence set forth in SEQ ID NO: 3 or an equivalent variant thereof;
(b) the CDR1 comprises or is the sequence set forth in SEQ ID NO: 5 or an equivalent variant thereof, the CDR2 comprises or is the sequence set forth in SEQ ID NO: 6 or an equivalent variant thereof, the CDR3 comprises or is the sequence set forth in SEQ ID NO: 7 or an equivalent variant thereof;
(c) the CDR1 comprises or is the sequence set forth in SEQ ID NO: 9 or an equivalent variant thereof, the CDR2 comprises or is the sequence set forth in SEQ ID NO: 10 or an equivalent variant thereof, the CDR3 comprises or is the sequence set forth in SEQ ID NO: 11 or an equivalent variant thereof;
(d) the CDR1 comprises or is the sequence set forth in SEQ ID NO: 13 or an equivalent variant thereof, the CDR2 comprises or is the sequence set forth in SEQ ID NO: 14 or an equivalent variant thereof, the CDR3 comprises or is the sequence set forth in SEQ ID NO: 15 or an equivalent variant thereof; or
(e) the CDR1 comprises or is the sequence set forth in SEQ ID NO: 17 or an equivalent variant thereof, the CDR2 comprises or is the sequence set forth in SEQ ID NO: 18 or an equivalent variant thereof, the CDR3 comprises or is the sequence set forth in SEQ ID NO: 19 or an equivalent variant thereof;

and wherein the second antigen is DDR2 and the second binding moiety comprises a nano-antibody, the nano-antibody comprises a CDR1, a CDR2, and a CDR3, and:
(a) the CDR1 comprises or is the sequence set forth in SEQ ID NO: 1 or an equivalent variant thereof, the CDR2 comprises or is the sequence set forth in SEQ ID NO: 2 or an equivalent variant thereof, the CDR3 comprises or is the sequence set forth in SEQ ID NO: 3 or an equivalent variant thereof;
(b) the CDR1 comprises or is the sequence set forth in SEQ ID NO: 5 or an equivalent variant thereof, the CDR2 comprises or is the sequence set forth in SEQ ID NO: 6 or an equivalent variant thereof, the CDR3 comprises or is the sequence set forth in SEQ ID NO: 7 or an equivalent variant thereof;
(c) the CDR1 comprises or is the sequence set forth in SEQ ID NO: 9 or an equivalent variant thereof, the CDR2 comprises or is the sequence set forth in SEQ ID NO: 10 or an equivalent variant thereof, the CDR3 comprises or is the sequence set forth in SEQ ID NO: 11 or an equivalent variant thereof;
(d) the CDR1 comprises or is the sequence set forth in SEQ ID NO: 13 or an equivalent variant thereof, the CDR2 comprises or is the sequence set forth in SEQ ID NO: 14 or an equivalent variant thereof, the CDR3 comprises or is the sequence set forth in SEQ ID NO: 15 or an equivalent variant thereof; or
(e) the CDR1 comprises or is the sequence set forth in SEQ ID NO: 17 or an equivalent variant thereof, the CDR2 comprises or is the sequence set forth in SEQ ID NO: 18 or an equivalent variant thereof, the CDR3 comprises or is the sequence set forth in SEQ ID NO: 19 or an equivalent variant thereof;

In some embodiments, the nano-antibody comprises or is the sequence selected from any one of SEQ ID NO: 4, SEQ ID NO: 8, SEQ ID NO: 12, SEQ ID NO: 16, and SEQ ID NO: 20, and an equivalent variant of each thereof.

In some embodiments, the nano-antibody comprised in the first binding moiety is different from the nano-antibody comprised in the second binding moiety.

In some embodiments, the present invention provides a bispecific antibody, comprising a first binding moiety binding to a first antigen and a second binding moiety binding to a second antigen, wherein the first binding moiety comprises a nano-antibody comprising or being the sequence of SEQ ID NO: 4, and wherein the second binding moiety comprises a nano-antibody comprising or being the sequence of SEQ ID NO: 12.

In some embodiments, the first binding moiety and the second binding moiety are connected through a linker. The linker is preferably a flexible linker such that it does not limit the effector molecule or polypeptide in a single undesired conformation. The linker is preferably composed primarily of amino acids having small side chains, such as glycine, alanine and serine, to provide said flexibility. In some embodiments, the linker comprises glycine-serine polymers, including but not limited to (GS)ₙ, (GGS)ₙ, (GGGS)ₙ (SEQ ID NO: 26) and combinations thereof, where n is an integer of at least 1. In some embodiments, the sequence of the linker is (GGGS)₃ (SEQ ID NO: 27). Other different linkers can also be used, including a variety of flexible linker designs that have been successfully used to link different antibody variable regions. The size and sequence composition of the linker can be determined by conventional computer modeling and techniques.

### Antibody-Drug Conjugate

Antibody-drug conjugates (ADCs) are a new class of highly potent biopharmaceutical drug designed as a targeted therapy, in particular for the treatment of cancer. ADCs are complex molecules composed of an antibody (a whole mAb or an antibody fragment) linked, via a stable, chemical, linker that may possess labile bonds, to a biologically active drug or cytotoxic compound. By combining the unique targeting capabilities of antibodies with the cell-killing ability of cytotoxic drugs, antibody-drug conjugates allow sensitive discrimination between healthy and diseased tissue, based on expression of the antibody antigen. This means that, in contrast to traditional chemotherapeutic agents, antibody-drug conjugates actively target and attack cancer cells, so that healthy cells with little or no antigen expression are less severely affected.

Accordingly, in another aspect, the present invention provides an antibody-drug conjugate, comprising an antibody targeting DDR2, a drug, and a linker connecting the antibody and the drug, wherein the antibody targeting DDR2 comprises or is any one of the nano-antibodies described herein.

In some embodiments, the drug used for conjugation to the antibody of the present invention is a cytotoxic agent, a chemotherapeutic agent, or a therapeutic radioisotope.

In some embodiments, the cytotoxic agent is a small molecular weight toxin, a peptide toxin, or a protein toxin. In some embodiments, the toxin is selected from the group consisting of ricin, ricin A chain, ethidium bromide, colchicine, dihydroxy anthracin dione, diphtheria toxin, Pseudomonas exotoxin A, abrin, abrin A chain, modeccin A chain, alpha-sarcin, mitogellin, retstrictocin, curicin, and crotin.

In some embodiments, the chemotherapeutic agent is selected from the group consisting of alkylating agents, anthracycline antibiotics, antimetabolites, anti-microtubule/anti-mitotic agents, histone deacetylase inhibitors, kinase inhibitors, peptide antibiotics, platinum-based antineoplastics, topoisomerase inhibitors, DNA crosslinking agents, and cytotoxic antibiotics.

In some embodiments, the therapeutic radioisotope is selected from the group consisting of Lu-177, Y-90, Ac-225, As-211, Bi-212, Bi-213, Cs-137, Cr-51, Co-60, Dy-165, Er-169, Fm-255, Au-198, Ho-166, I-125, I-131, Ir-192, Fe-59, Pb-212, Mo-99, Pd-103, P-32, K-42, Re-186, Re-188, Sm-153, Ra-223, Ru-106, Na-24, Sr-89, Tb-149, Th-227, Xe-133, Yb-169, and Yb-177.

In some embodiments, the present invention provides an antibody-drug conjugate, comprising an antibody targeting DDR2, a drug (preferably MMAE, calicheamicin, IR700, SG3199, DM1, DXd, MMAF, PE-38, SN-38, or yttrium-90), and a linker connecting the antibody and the drug, wherein the antibody comprises or is a nano-antibody, the nano-antibody comprises a CDR1, a CDR2, and a CDR3, and:
(a) the CDR1 comprises or is the sequence set forth in SEQ ID NO: 1 or an equivalent variant thereof, the CDR2 comprises or is the sequence set forth in SEQ ID NO: 2 or an equivalent variant thereof, the CDR3 comprises or is the sequence set forth in SEQ ID NO: 3 or an equivalent variant thereof;
(b) the CDR1 comprises or is the sequence set forth in SEQ ID NO: 5 or an equivalent variant thereof, the CDR2 comprises or is the sequence set forth in SEQ ID NO: 6 or an equivalent variant thereof, the CDR3 comprises or is the sequence set forth in SEQ ID NO: 7 or an equivalent variant thereof;
(c) the CDR1 comprises or is the sequence set forth in SEQ ID NO: 9 or an equivalent variant thereof, the CDR2 comprises or is the sequence set forth in SEQ ID NO: 10 or an equivalent variant thereof, the CDR3 comprises or is the sequence set forth in SEQ ID NO: 11 or an equivalent variant thereof;
(d) the CDR1 comprises or is the sequence set forth in SEQ ID NO: 13 or an equivalent variant thereof, the CDR2 comprises or is the sequence set forth in SEQ ID NO: 14 or an equivalent variant thereof, the CDR3 comprises or is the sequence set forth in SEQ ID NO: 15 or an equivalent variant thereof; or
(e) the CDR1 comprises or is the sequence set forth in SEQ ID NO: 17 or an equivalent variant thereof, the CDR2 comprises or is the sequence set forth in SEQ ID NO: 18 or an equivalent variant thereof, the CDR3 comprises or is the sequence set forth in SEQ ID NO: 19 or an equivalent variant thereof.

### siRNA Conjugate

In another aspect, the present invention provides an siRNA conjugate targeting a DDR2 positive cell, comprising: (i) an antibody targeting DDR2, (ii) an siRNA that inhibits the expression of a survival gene in a DDR2 positive cell, and (iii) a linker positioned between the nano-antibody and the siRNA, wherein antibody targeting DDR2 comprises or is any one of the nano-antibodies described herein.

As the pharmacological part of the siRNA conjugate disclosed herein, the siRNA portion that inhibits the expression of a survival gene in a DDR2 positive cell is a double stranded RNA molecule that, after being taken up by target cells, specifically targets the degradation of mRNA of a specific survival gene through RNAi mechanism, thereby inhibiting the expression of a survival gene in a DDR2 positive cell.

The method of designing a siRNA targeting a target gene is a conventional technique in the art. For example, there are multiple siRNA design websites that can provide online design services (such as DSIR: http://biodev.extra.cea.fr/DSIR/DSIR.html or siDirect version 2.0: http://sidirect2.rnai.jp/). These design websites can simultaneously obtain multiple representative siRNA sequences targeting different fragments of the target gene based on its mRNA. These representative siRNAs can be sorted based on multiple options, such as thermodynamics, advanced structure, etc. Candidate siRNA sequences can be further screened (manually or automatically) to exclude sequences containing SNP sites or binding non-open reading frame fragments. In some embodiments, overlapping or coinciding results from different online design websites are selected as candidate siRNA sequences. Candidate siRNA sequences can also be analyzed for siRNA sequences having potential high off-target property using a off-target predictiing software, such as http://rnai.cs.unm.edu/rnai/off-target.

In some embodiments, the siRNA sequence of the siRNA portion targets the a survival gene of a DDR2 positive cell. For example, the siRNA sequence targets one or more fragments of one or more of the *polr2a, polr2b, dkc1, cenpe, eif-3b* genes. In some embodiments, when siRNA sequences are delivered to cells, they bind to the target gene through the RNAi pathway, resulting in inhibition of the expression of the target gene. In some embodiments, inhibition of target gene expression leads to apoptosis, death, and/or proliferation inhibition of DDR2 positive cells. In some embodiments, apoptosis, death, and/or proliferation inhibition of DDR2 positive cells mediate the treatment or cure of diseases. Suitable survival genes include but are not limited to one or more of the *polr2a, polr2b, dkc1, cenpe, eif-3b,* and *surviving* genes. SiRNA sequences targeting survival genes can be obtained and tested using the methods described above.

In some embodiments, the present invention provides an siRNA conjugate targeting a DDR2 positive cell, comprising: (i) an antibody targeting DDR2, (ii) an siRNA that inhibits the expression of a survival gene in a DDR2 positive cell, and (iii) a linker positioned between the nano-antibody and the siRNA, wherein antibody targeting DDR2 comprises or is a nano-antibody, wherein the nano-antibody comprises a CDR1, a CDR2, and a CDR3, and:
(a) the CDR1 comprises or is the sequence set forth in SEQ ID NO: 1 or an equivalent variant thereof, the CDR2 comprises or is the sequence set forth in SEQ ID NO: 2 or an equivalent variant thereof, the CDR3 comprises or is the sequence set forth in SEQ ID NO: 3 or an equivalent variant thereof;
(b) the CDR1 comprises or is the sequence set forth in SEQ ID NO: 5 or an equivalent variant thereof, the CDR2 comprises or is the sequence set forth in SEQ ID NO: 6 or an equivalent variant thereof, the CDR3 comprises or is the sequence set forth in SEQ ID NO: 7 or an equivalent variant thereof;
(c) the CDR1 comprises or is the sequence set forth in SEQ ID NO: 9 or an equivalent variant thereof, the CDR2 comprises or is the sequence set forth in SEQ ID NO: 10 or an equivalent variant thereof, the CDR3 comprises or is the sequence set forth in SEQ ID NO: 11 or an equivalent variant thereof;
(d) the CDR1 comprises or is the sequence set forth in SEQ ID NO: 13 or an equivalent variant thereof, the CDR2 comprises or is the sequence set forth in SEQ ID NO: 14 or an equivalent variant thereof, the CDR3 comprises or is the sequence set forth in SEQ ID NO: 15 or an equivalent variant thereof; or
(e) the CDR1 comprises or is the sequence set forth in SEQ ID NO: 17 or an equivalent variant thereof, the CDR2 comprises or is the sequence set forth in SEQ ID NO: 18 or an equivalent variant thereof, the CDR3 comprises or is the sequence set forth in SEQ ID NO: 19 or an equivalent variant thereof.

In some embodiments, an antibody comprising any one of the nano-antibodies described herein may be an antibody comprising two intact heavy chains, each nano-antibody constituting a variable region of each intact heavy chain, and each intact heavy chain further comprising a constant region of the heavy chain, such as a human heavy chain constant region. In some embodiments, an antibody comprising any of the nano-antibodies described herein may comprises two of the nano-antibodies.

In some embodiments, the Fc region of the heavy chain constant region is mutated. In some embodiments, the Fc region is mutated to reduce antibody mediated cytotoxicity (ADCC). Exemplary Fc region mutations can be L234A, L235A, G237A, and P329G, with the position numbering according to the EU index.

### Pharmaceutical Composition

In another aspect, the present invention provides a pharmaceutical composition comprising a therapeutically effective amount of any one of the antibody, nucleotide sequence, vector, host cell, immune cell, or conjugate described above, and a pharmaceutically acceptable carrier.

In any of the above aspects, the pharmaceutical composition is for use in treating and/or preventing a disease mediated by abnormal DDR2 expression, such as cancer or inflammatory disease, such as pulmonary fibrosis and retinal vasculopathy.

**To** prepare a pharmaceutical or sterile composition, the drug is mixed with a pharmaceutically acceptable carrier or excipient. Formulations in the form of such as freeze-dried powder, slurry, aqueous solution, or suspension can be prepared by mixing with physiologically acceptable carriers, excipients, or stabilizers. Pharmaceutically acceptable carriers are well-known in the art. It is known in the art how to prepare aqueous compositions containing active ingredients. Generally, these compositions are prepared into injections or sprays, such as liquid solutions or suspensions; It can also be prepared into a solid form suitable for preparation into a solution or suspension before injection or spray.

### Reagent for Imaging and/or Diagnosis

In another aspect, the present invention provides a reagent for imaging and/or diagnosis, comprising any one of the nano-antibodies described above and a detectable label.

Accordingly, the present invention provides use of any one of the nano-antibodies described above in the manufacture of a reagent for imaging and/or diagnosis.

Accordingly, the present invention provides any one of the nano-antibodies described above for use in imaging and/or diagnosis.

The above reagent for imaging and/or diagnosis can be used for disease diagnosis or lesion imaging, such as pulmonary fibrosis imaging and retinal neovascularization imaging. The reagent can also be used as an imaging reagent for intraoperative imaging navigation, such as optical imaging of lesions, to guide surgeons for surgical navigation and achieve precise resection of lesions.

The detectable label included in the reagent for imaging and/or diagnosis of the present invention may be, for example, a fluorescent label, a chemiluminescent label, a paramagnetic label, a radioisotopic label, or an enzyme label.

The choice of label depends on the means of detection. For example, a fluorescent label (such as indocyanine green (ICG), a rare earth chelate (e.g., a europium chelate), a fluorescein-type label (e.g., fluorescein, fluorescein isothiocyanate, 5-carboxyfluorescein, 6-carboxyfluorescein, dichlorotriazinylamine fluorescein), a rhodamine- type label (e.g., ALEXA568(Invitrogen), or dansyl chloride), VIVOTAG 680 XLFLUOROCHROMETM(Perkin Elmer), phycoerythrin; umbelliferone, Lissamine; a cyanine; a phycoerythrin, Texas Red, BODIPY(Invitrogen) or an analogue thereof, is suitable for optical detection.

Chemoluminescent labels may also be employed (e.g., luminol, luciferase, luciferin, and aequorin). Such diagnosis and detection can also be accomplished by coupling the nano-antibody of the present invention to detectable substances including, but not limited to, various enzymes, enzymes including, but not limited to, horseradish peroxidase, alkaline phosphatase, beta-galactosidase, or acetylcholinesterase, or to prosthetic group complexes such as, but not limited to, streptavidin/biotin and avidin/biotin.

Paramagnetic labels and radioisotopic labels can also be employed, and are preferably detected using Positron Emission Tomography (PET) or Single-PhotonEmission Computed Tomography (SPECT). Radiolabels include, but are not limited to, bismuth (²¹³Bi), carbon (¹¹C, ¹³C, ¹⁴C), chromium (⁵¹Cr), cobalt (⁵⁷Co, ⁶⁰Co), copper (⁶⁴Cu), dysprosium (¹⁶⁵Dy), erbium (¹⁶⁹Er), fluorine (¹⁸F), gadolinium (¹⁵³Gd, ¹⁵⁹Gd), gallium (⁶⁸Ga, ⁶⁷Ga), germanium (⁶⁸Ge), gold (¹⁹⁸Au), holmium (¹⁶⁶Ho), hydrogen (³H), indium (¹¹¹In, ¹¹²In, ¹¹³In, ¹¹⁵In), iodine (¹²¹I, ¹²³I, ¹²⁵I, ¹³¹I), iridium (¹⁹²Ir), iron (⁵⁹Fe), krypton (^{81m}Kr), lanthanium (¹⁴⁰La), lutelium (¹⁷⁷Lu), manganese (⁵⁴Mn), molybdenum (⁹⁹Mo), nitrogen (¹³N, ¹⁵N), oxygen (¹⁵O), palladium (¹⁰³Pd), phosphorus (³²P), potassium (⁴²K), praseodymium (¹⁴²Pr), promethium (¹⁴⁹Pm), rhenium (¹⁸⁶Re, ¹⁸⁸Re), rhodium (¹⁰⁵Rh), rubidium (⁸¹Rb, ⁸²Rb), ruthenium (⁸²Ru, ⁹⁷Ru), samarium (¹⁵³Sm), scandium (⁴⁷Sc), selenium (⁷⁵Se), sodium (²⁴Na), strontium (⁸⁵Sr, ⁸⁹Sr, ⁹²Sr), sulfur (³⁵S), technetium (⁹⁹Tc), thallium (²⁰¹Tl), tin (¹¹³Sn, ¹¹⁷Sn), xenon (¹³³Xe), ytterbium (¹⁶⁹Yb, ¹⁷⁵Yb, ¹⁷⁷Yb), yttrium (⁹⁰Y), and zinc (⁶⁵Zn); positron emitting metals using various positron emission tomographies, and nonradioactive paramagnetic metal ions (such as paramagnetic ions of Aluminum (Al), Barium (Ba), Calcium (Ca), Cerium (Ce), Dysprosium (Dy), Erbium (Er), Europium (Eu), Gandolinium (Gd), Holmium (Ho), Iridium (Ir), Lithium (Li), Magnesium (Mg), Manganese (Mn), Molybdenum (M), Neodymium (Nd), Osmium (Os), Oxygen (O), Palladium (Pd), Platinum (Pt), Rhodium (Rh), Ruthenium (Ru), Samarium (Sm), Sodium (Na), Strontium (Sr), Terbium (Tb), Thulium (Tm), Tin (Sn), Titanium (Ti), Tungsten (W), and Zirconium (Zr), and particularly, Co⁺², CR⁺², Cr⁺³, Cu⁺², Fe⁺², Fe⁺³, Ga⁺³, Mn⁺³, Ni⁺², Ti⁺³, V⁺, and V⁺⁴). Methods for preparing radiolabeled amino acids and related peptide derivatives are known in the art. For example, a radioisotope may be conjugated by a chloramine-T method.

In some embodiments, the present invention provides a reagent for imaging and/or diagnosis, comprising any one of the nano-antibodies described above and a detectable label connected with the nano-antibody, wherein the detectable label is a fluorescent label. In some embodiments, the fluorescent label is indocyanine green (ICG).

In some embodiments, the present invention provides a reagent for imaging and/or diagnosis, comprising any one of the nano-antibodies described above and a detectable label connected with the nano-antibody, wherein the detectable label is a fluorescent label, preferably indocyanine green (ICG), and wherein the nano-antibody comprises a CDR1, a CDR2, and a CDR3, and:
(a) the CDR1 comprises or is the sequence set forth in SEQ ID NO: 1 or an equivalent variant thereof, the CDR2 comprises or is the sequence set forth in SEQ ID NO: 2 or an equivalent variant thereof, the CDR3 comprises or is the sequence set forth in SEQ ID NO: 3 or an equivalent variant thereof;
(b) the CDR1 comprises or is the sequence set forth in SEQ ID NO: 5 or an equivalent variant thereof, the CDR2 comprises or is the sequence set forth in SEQ ID NO: 6 or an equivalent variant thereof, the CDR3 comprises or is the sequence set forth in SEQ ID NO: 7 or an equivalent variant thereof;
(c) the CDR1 comprises or is the sequence set forth in SEQ ID NO: 9 or an equivalent variant thereof, the CDR2 comprises or is the sequence set forth in SEQ ID NO: 10 or an equivalent variant thereof, the CDR3 comprises or is the sequence set forth in SEQ ID NO: 11 or an equivalent variant thereof;
(d) the CDR1 comprises or is the sequence set forth in SEQ ID NO: 13 or an equivalent variant thereof, the CDR2 comprises or is the sequence set forth in SEQ ID NO: 14 or an equivalent variant thereof, the CDR3 comprises or is the sequence set forth in SEQ ID NO: 15 or an equivalent variant thereof; or
(e) the CDR1 comprises or is the sequence set forth in SEQ ID NO: 17 or an equivalent variant thereof, the CDR2 comprises or is the sequence set forth in SEQ ID NO: 18 or an equivalent variant thereof, the CDR3 comprises or is the sequence set forth in SEQ ID NO: 19 or an equivalent variant thereof.

In some embodiments, the present invention provides a reagent for imaging and/or diagnosis, comprising any one of the nano-antibodies described above and a detectable label connected with the nano-antibody, wherein the detectable label is a radioisotope. In some embodiments, the radioisotope is ⁶⁸Ga or ⁶⁴Cu.

In some embodiments, the present invention provides a reagent for imaging and/or diagnosis, comprising any one of the nano-antibodies described above and a detectable label connected with the nano-antibody, wherein the detectable label is a radioisotope, preferably ⁶⁸Ga or ⁶⁴Cu, and wherein the nano-antibody comprises a CDR1, a CDR2, and a CDR3, and:
(a) the CDR1 comprises or is the sequence set forth in SEQ ID NO: 1 or an equivalent variant thereof, the CDR2 comprises or is the sequence set forth in SEQ ID NO: 2 or an equivalent variant thereof, the CDR3 comprises or is the sequence set forth in SEQ ID NO: 3 or an equivalent variant thereof;
(b) the CDR1 comprises or is the sequence set forth in SEQ ID NO: 5 or an equivalent variant thereof, the CDR2 comprises or is the sequence set forth in SEQ ID NO: 6 or an equivalent variant thereof, the CDR3 comprises or is the sequence set forth in SEQ ID NO: 7 or an equivalent variant thereof;
(c) the CDR1 comprises or is the sequence set forth in SEQ ID NO: 9 or an equivalent variant thereof, the CDR2 comprises or is the sequence set forth in SEQ ID NO: 10 or an equivalent variant thereof, the CDR3 comprises or is the sequence set forth in SEQ ID NO: 11 or an equivalent variant thereof;
(d) the CDR1 comprises or is the sequence set forth in SEQ ID NO: 13 or an equivalent variant thereof, the CDR2 comprises or is the sequence set forth in SEQ ID NO: 14 or an equivalent variant thereof, the CDR3 comprises or is the sequence set forth in SEQ ID NO: 15 or an equivalent variant thereof; or
(e) the CDR1 comprises or is the sequence set forth in SEQ ID NO: 17 or an equivalent variant thereof, the CDR2 comprises or is the sequence set forth in SEQ ID NO: 18 or an equivalent variant thereof, the CDR3 comprises or is the sequence set forth in SEQ ID NO: 19 or an equivalent variant thereof.

In some embodiments, the nano-antibody comprises or is the sequence selected from any one of SEQ ID NO: 4, SEQ ID NO: 8, SEQ ID NO: 12, SEQ ID NO: 16, and SEQ ID NO: 20 and an equivalent variant of each thereof.

### Method and Use

In another aspect, the present invention provides a method for treating and/or preventing a disease mediated by abnormal DDR2 expression, comprising administering to a subject an effective amount of any one of the antibody, polynucleotide, vector, host cell, immune cell, or conjugate described above.

Accordingly, in another aspect, the present invention provides use of any one of the antibody, polynucleotide, vector, host cell, immune cell, or conjugate described above in the manufacture of a medicament for treating and/or preventing a disease mediated by abnormal DDR2 expression.

Accordingly, in another aspect, the present invention provides any one of the antibody, polynucleotide, vector, host cell, immune cell, or conjugate described above for use in treating and/or preventing a disease mediated by abnormal DDR2 expression.

In aother aspect, the present invention provides a method for the diagnosis of a disease mediated by abnormal DDR2 expression in a subject, comprising administering to the subject an effective amount of any one of the reagent for imaging and/or diagnosis described above. In some embodiments, the method comprises: administering to the subject an effective amount of any one of the reagent for imaging and/or diagnosis described above; detecting and reading a signal generated by the reagent for imaging and/or diagnosis; and determining that the subject has or is at risk of having a disease mediated by abnormal DDR2 expression based on the strength of the signal. In some embodiments, the method comprises determining whether the signal exceeds a predetermined threshold, and and when the signal exceeds the predetermined threshold, determining that the subject is suffering from a disease mediated by abnormal DDR2 expression. In some embodiments, the threshold is a median level from a subject not suffering from the disease.

Accordingly, in another aspect, the present invention provides use of any one of the reagent for imaging and/or diagnosis described above in the manufacture of a kit for the diagnosis of a disease mediated by abnormal DDR2 expression in a subject.

Accordingly, in another aspect, the present invention provides any one of the reagent for imaging and/or diagnosis described above for use in the diagnosis of a disease mediated by abnormal DDR2 expression in a subject.

In some embodiments, the present invention provides a method for treating a disease mediated by abnormal DDR2 expression in a subject, comprising: (a) administering to the subject an effective amount of any one of the reagent for imaging and/or diagnosis described above; (b) detecting and reading a signal generated by the reagent for imaging and/or diagnosis; (c) determining whether the signal exceeds a predetermined threshold, and and when the signal exceeds the predetermined threshold, determining that the subject is suffering from a disease mediated by abnormal DDR2 expression, preferablly wherein the threshold is a median level from a subject not suffering from the disease; and (e) administering to the subject determined to be suffering from the disease a therapy that reduces DDR2 activity and/or expression, such as anticancer therapy or anti-inflammatory therapy, such as anti-fibrosis therapy. In a preferred embodiment, the disease mediated by abnormal DDR2 expression is pulmonary fibrosis, particularly idiopathic pulmonary fibrosis. In a preferred embodiment, the anti-fibrosis therapy is Pirfenidone and/or Nintedanib.

Suitable routes of administration include parenteral administration (for example, intramuscular, intravenous or subcutaneous administration) and oral administration. Other conventional administration methods include administration by tracheal intubation, oral ingestion, inhalation, topical application or transdermal, subcutaneous, intraperitoneal, intraarterial.

The appropriate dose is determined by the clinician, for example, using parameters or factors known or suspected to affect the treatment or expected to affect the treatment in the art. Generally, the starting dose is slightly lower than the optimal dose, and thereafter a small increase until the desired or optimal effect relative to any adverse side effects is achieved. Important diagnostic measures include measuring, for example, inflammatory symptoms or the level of inflammatory cytokines produced.

### Sequence Listing

| SEQ ID NO: | Description | Sequence |
|---|---|---|
| 1 | 1A1-CDR1 | GRIFSINV |
| 2 | 1A1-CDR2 | INTRGGST |
| 3 | 1A1-CDR3 | AASRKGYPSTRSDPYDY |
| 4 | 1A1-VHH | EVQLVESGGGLVQPGGSLRLSCAASGRIFSINVMGWF RQAPGKGRELVAAINTRGGSTYYPDSVEGRFTISRDNA KRMVYLQMNSLRAEDTAVYYCAASRKGYPSTRSDPYD YWGQGTQVTVSS |
| 5 | 1A5-CDR1 | GITLSLYI |
| 6 | 1A5-CDR2 | ISRTGGST |
| 7 | 1A5-CDR3 | AADFYGNATLGYNY |
| 8 | 1A5-VHH | EVQLVESGGGLVQPGGSLRLSCAASGITLSLYIMGWFR QAPGKGRELVAAISRTGGSTYYPDSVEGRFTISRDNAK RMVYLQMNSLRAEDTAVYYCAADFYGNATLGYNYWG QGTQVTVSS |
| 9 | 1A12-CDR1 | GYILSPNV |
| 10 | 1A12-CDR2 | ISWGDGRT |
| 11 | 1A12-CDR3 | AASRKGYPSTRSDPYNY |
| 12 | 1A12-VHH | EVQLVESGGGLVQPGGSLRLSCAASGYILSPNVMGWF RQAPGKGRELVAAISWGDGRTYYPDSVEGRFTISRDN AKRMVYLQMNSLRAEDTAVYYCAASRKGYPSTRSDPY NYWGQGTQVTVSS |
| 13 | 1B2-CDR1 | GYIFSIYA |
| 14 | 1B2-CDR2 | ISWGDGST |
| 15 | 1B2-CDR3 | AAAIKGRGWTQLGYNY |
| 16 | 1B2-VHH | EVQLVESGGGLVQPGGSLRLSCAASGYIFSIYAMGWFR QAPGKGRELVARISWGDGSTWYPDSVEGRFTISRDNA KRMVYLQMNSLRAEDTAVYYCAAAIKGRGVVTQLGYN YWGQGTQVTVSS |
| 17 | 1B3-CDR1 | GDTFSYYV |
| 18 | 1B3-CDR2 | ISRTGGST |
| 19 | 1B3-CDR3 | AADPYISGTLGYNY |
| 20 | 1B3-VHH | EVQLVESGGGLVQPGGSLRLSCAASGDTFSYYVMGW FRQAPGKGRELVAAISRTGGSTYYPDSVEGRFTISRDN AKRMVYLQMNSLRAEDTAVYYCAADPYISGTLGYNYW GQGTQVTVSS |
| 21 | 1A1-VHH DNA | GAGGTGCAGCTGGTGGAGAGCGGTGGTGGTCTCGT GCAGCCCGGCGGTAGTCTGCGCCTCAGCTGTGCCG CCAGCGGTCGCATCTTTAGCATCAATGTGATGGGCT GGTTTCGCCAAGCCCCCGGCAAAGGTCGCGAACTG GTGGCCGCCATTAACACCCGCGGTGGCAGCACCTAT TACCCAGATAGCGTGGAAGGCCGCTTCACCATCAGC CGCGATAACGCCAAGCGCATGGTGTATCTGCAGATG AACAGTCTGCGCGCCGAGGACACCGCCGTGTATTAT TGTGCCGCCAGCCGCAAAGGCTATCCAAGCACCCG CAGCGATCCATACGATTACTGGGGCCAAGGCACCCA AGTGACCGTGAGCAGC |
| 22 | 1A5-VHH DNA | GAGGTGCAGCTGGTGGAGAGCGGTGGTGGTCTCGT GCAGCCCGGCGGTAGTCTGCGCCTCAGCTGTGCCG CCAGCGGTATCACCCTTAGCCTGTATATCATGGGCTG GTTTCGCCAAGCCCCCGGCAAAGGTCGCGAACTGG TGGCCGCCATTTCCCGCACGGGCGGTAGCACCTACT ACCCAGATAGCGTGGAAGGCCGCTTCACCATCAGCC GCGATAACGCCAAGCGCATGGTGTATCTGCAGATGA ACAGTCTGCGCGCCGAGGACACCGCCGTGTATTATT GTGCCGCCGATTTTTATGGCAACGCCACCCTGGGCT ACAATTACTGGGGCCAAGGCACCCAAGTGACCGTGA GCAGC |
| 23 | 1A12-VHH DNA | GAGGTGCAGCTGGTGGAGAGCGGTGGTGGTCTCGT GCAGCCCGGCGGTAGTCTGCGCCTCAGCTGTGCCG CCAGCGGTTATATCCTTAGCCCAAATGTGATGGGCTG GTTTCGCCAAGCCCCCGGCAAAGGTCGCGAACTGG TGGCCGCCATTAGCTGGGGCGATGGCCGCACCTATT ACCCAGATAGCGTGGAAGGCCGCTTCACCATCAGCC GCGATAACGCCAAGCGCATGGTGTATCTGCAGATGA ACAGTCTGCGCGCCGAGGACACCGCCGTGTATTATT GTGCCGCCAGCCGCAAAGGCTATCCAAGCACCCGC AGCGATCCATACAATTACTGGGGCCAAGGCACCCAA GTGACCGTGAGCAGC |
| 24 | 1B2-VHH DNA | GAGGTGCAGCTGGTGGAGAGCGGTGGTGGTCTCGT GCAGCCCGGCGGTAGTCTGCGCCTCAGCTGTGCCG CCAGCGGTTATATCTTTAGCATCTATGCCATGGGCTG GTTTCGCCAAGCCCCCGGCAAAGGTCGCGAACTGG TGGCCCGCATTAGCTGGGGCGATGGCAGCACCTGG TACCCAGATAGCGTGGAAGGCCGCTTCACCATCAGC CGCGATAACGCCAAGCGCATGGTGTATCTGCAGATG AACAGTCTGCGCGCCGAGGACACCGCCGTGTATTAT TGTGCCGCCGCCATCAAAGGCCGCGGCGTGGTGAC CCAGCTGGGCTACAATTACTGGGGCCAAGGCACCCA AGTGACCGTGAGCAGC |
| 25 | 1B3-VHH DNA | GAGGTGCAGCTGGTGGAGAGCGGTGGTGGTCTCGT GCAGCCCGGCGGTAGTCTGCGCCTCAGCTGTGCCG CCAGCGGTGATACCTTTAGCTATTATGTGATGGGCTG GTTTCGCCAAGCCCCCGGCAAAGGTCGCGAACTGG TGGCCGCCATTTCCCGCACGGGCGGTAGCACCTACT ACCCAGATAGCGTGGAAGGCCGCTTCACCATCAGCC GCGATAACGCCAAGCGCATGGTGTATCTGCAGATGA ACAGTCTGCGCGCCGAGGACACCGCCGTGTATTATT GTGCCGCCGATCCATATATCAGCGGCACCCTGGGCT ACAATTACTGGGGCCAAGGCACCCAAGTGACCGTGA GCAGC |
| 26 | linker | GGGS |
| 27 | linker | GGGSGGGSGGGS |

### Examples

### Example 1: Screening of antibodies

Anti-DDR2 heavy chain antibodies were screened from alpacas immunized with human DDR2 extracellular segment (UniProtKB/Swiss-Prot: Q16832.2 aa 22 to aa 399). Then, the antibodies were sequenced to confirm their VHH portions. Five nano-antibodies were obtained and named 1A1, 1A5, 1A12, 1B2, and 1B3, resepetively, with the sequences shown in the table above, and were used for further characterization and experimentation.

### Example 2: Expression and purification of five screened DDR2 nano-antibodies

### Experimental procedure:

### I. Transformation

A tube containing 100 µL BL21 (DE3) competent cells was taken and placed on ice. 1 µL of plasmids (expression vectors of the 5 screened DDR2 nano-antibodies) was added. The tube was flicked on the bottom, placed on ice for 25-30 minutes, subjected to 42°C water bath for 45 seconds, and then quickly put back to the ice surface for two minutes. 500 µL of non-resistant LB medium was added, followed by treatment at 37°C and 250rpm for 1 hour. Centrifugation was performed at 3000 rpm for 1 minute, and 100 µL of supernatant was kept, and evenly mixed by blowing and beating, and coated to LB agar plate (LBA+) containing ampicillin, which was incubated in a 37°C incubator overnight.

### II. Preservation and amplification of bacterial strains

1. Preservation: The monoclonal antibodies were picked from the plate and added to 5-10ml of LBA+ liquid culture medium, followed by treatment at 37°C and 250rpm overnight. The next day, 750 µL of bacterial solution was taken and added to 250 µL of 80% LB glycerol. The fragment, vector, strain name, and date were written down. The solution was uniformly mixed, and stored at -20°C/-80°C (after 3-4 months of preservation with glycerol, the bacteria need to be coated to plate to be activated, and then subjected to another preservation).
2. Amplification:

Day 1: 10 µL of bacteria in glycerol was taken and added to 10ml of LBA+ liquid culture medium (50ml centrifuge tube), forming a total of 15 tubes, followed by treatment at 37°C and 250rpm overnight. 2L of LBA+liquid culture medium was prepared and divided into 8 1L conical flasks, 250ml/flask, followed by treatment at 121°C for 16 minutes.

The next day: the overnight amplified bacterial solution was seeded into a 1L conical flask at a ratio of 6%, and simultaneously added with 250 µL of 100mg/ml Amp, followed by treatment at 37°C and 250rpm for 2-3 hours (OD value reached 0.8-1). Induction was performed by adding IPTG at a final concentration of 1mM, followed by treatment at 32°C and 200rpm for 5-6 hours. The bacterial cells were collected (10000rpm, 5min), and freezed and stored at -20°C.

### III. Protein extraction and purification

### 1. Protein extraction

The bacterial cells were taken out from -20°C and thawed at room temperature, resuspended in bacterial lysis buffer (500ml bacteria solution=50ml lysis buffer), sonicated for 5 minutes (450-500W, worked for 7 seconds and then stoped for 3 seconds); followed by treatment in a homogenizer at 1000Pa for 10 minutes; followed by treatment at 4°C and 12000rpm for 30 minutes. The supernatant was kept.

### 2. Protein purification

200 µL of magnetic beads (Kingsray, L00776) was sucked into a 1.5ml EP tube, followed by washing 5-6 times with WashBuffer (1ml wash solution per time). The wash buffer was discarded, and the magnetic beads were added to the bacterial supernatant, which was overturned and uniformly mixed for 1 hour at 4°C. The centrifuge tube was placed on a magnetic rack. When the supernatant became clear, it was transferred to a new centrifuge, which was added with new 200 µL magnetic beads, and was overturned and uniformly mixed for 1 hour at 4°C. The magnetic beads in the old tube were washed 5-6 times with WashBuffer, and then ElutionBuffer was added for elution for 3 times at a rate of 1ml/time, each time for 5 minutes, with being overturned and uniformly mixed for elution. Finally, all elution solutions were collected, and freezed and stored at -20°C.

### IV. Protein concentrating and fluid change

1. 20% alcohol from the ultrafiltration tube was poured out, followed by rinsing with double distilled water for three times, and adding 15ml of double distilled water, followed by treatment at 4°C and 4000rpm for 10-12 minutes. The double distilled water from the inner and outer tubes was poured out, followed by addiing eluent (not exceeding the inner tube scale line), and concentrating to about 500 µL.
2. 4.5ml of filtered PBS was added, followed by treatment at 4°C and 4000rpm for 30 minutes. The above steps were repeated for three times, and finally approximately 500-700 µL of the sample in PBS was aspirated and transfered to a 1.5ml EP tube. The ultrafiltration tube was rinsed, and the inner tube was added with 20% ethanol.
3. BCA method use used to detect protein concentration.

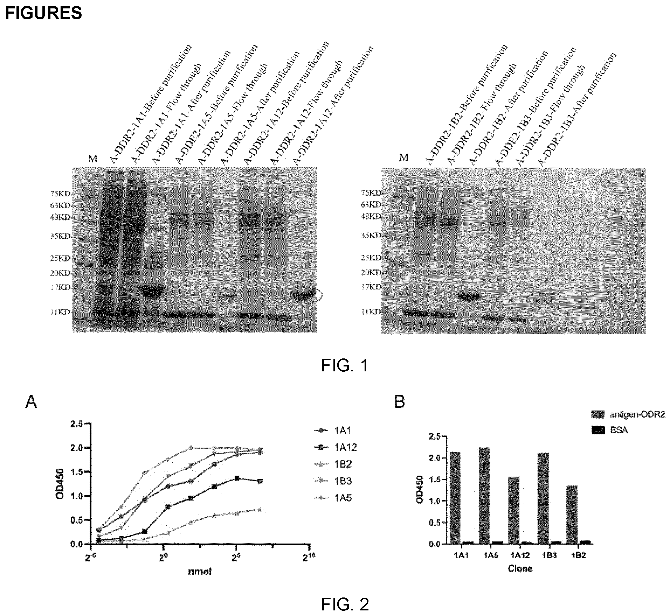

**Result analysis:** The SDS-PAGE results of 5 DDR2 nano-antibodies (1A1, 1A5, 1A12, 1B2, 1B3) expressed and purified are shown in Figure 1. The size of the antibody protein (circled) is approximately 15KD, which is consistent with expectation and the size is correct.

### Example 3: ELISA preliminary identification of DDR2 nano-antibodies binding to extracellular segment of DDR2 antigen

### Experimental procedure:

The Flag tag was fused into the nano-antibody gene coding sequence and five DDR2 antibodies with Flag tag were expressed and purified: 1A1, 1A5, 1A12, 1B2, 1B3; Then, the extracellular segment of DDR2 antigen was used to coat at 100ng/well → goat serum was used to block overnight → PBS was used to wash three times, anti-DDR2 (five purified DDR2 antibodies, all comprising Flag tag) 200ng/well, 37°C 1h → Anti Flag (Sigma, F1804-200UG, 1mg/ml) 1:2500, 37°C 1h → G&M (HRP) 1:5000, 37°C 30min (horseradish peroxidase labeled anti Flag antibody amplification signal, TMB staining) → TMB staining at 37°C 30min → 1M HCl for termination; A blank control was prepared simultaneously.

### Result analysis:

5 nano-antibodies that were expressed and purified and identified as correct in the previous step, along with the extracellular segment of DDR2 antigen and the negative control protein bovine serum albumin (BSA), were subjected to ELISA validation.

The analysis results of binding to the extracellular segment of DDR2 antigen are shown in Figure 2A. It can be seen that the five nano-antibodies 1A1, 1A5, 1A12, 1B2, and 1B3 can all show binding activity to DDR2 antigen.

The analysis results of binding to negative control protein bovine serum albumin are shown in Figure 2B. It can be seen that the five nano-antibodies 1A1, 1A5, 1A12, 1 B2, and 1B3 have no binding activity to BSA protein.

Therefore, it can be confirmed that the five nano-antibodies 1A1, 1A5, 1A12, 1 B2, and 1B3 have binding activity to DDR2 antigen, but do not bind to the negative control protein BSA.

### Example 4: Flow cytometry identification of DDR2 nano-antibodies binding to DDR2 antigen

### Experimental procedure:

A549 cells were infected with adenovirus overexpressing DDR2 antigen. DDR2 nano-antibodies (His tag was fused into the nano-antibody gene coding sequence, and five DDR2 nanobodies with His tag were expressed and purified) were incubated at 4°C 1h for 1 hour, followed by washing once with PBS, and Anti-His-AF488 was incubated as a secondary antibody at 4°C for half an hour, followed by washing once with PBS. Centrifugation was performed at 300g for 5 minutes, cells were collected and resuspended in 200ul. A ACEA NovoCyte next-generation intelligent flow cytometer was used for performing flow cytometry detection.

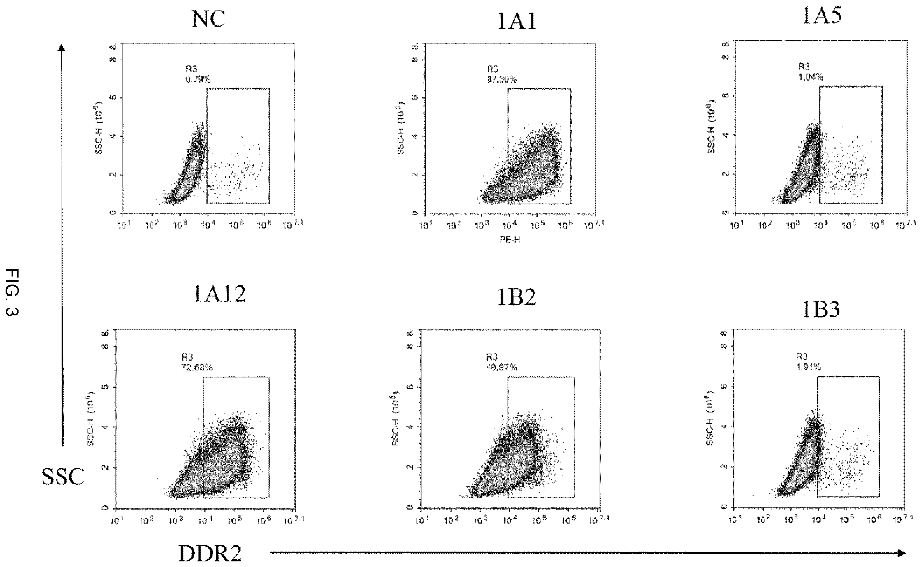

Result analysis: The analysis results of binding to DDR2 antigen are shown in Figure 3. It can be seen that the five nano-antibodies 1A1, 1A5, 1A12, 1B2, and 1B3 can all show binding activity to DDR2 antigen.

### Example 5: Immunofluorescence identification of DDR2 nano-antibodies binding to DDR2 antigen

### Experimental procedure:

Coating the plate with cells overexpressing DDR2 antigen → 4% paraformaldehyde fixation overnight → 5% milk powder blocking for 0.5 hours → DDR2 nano-antibody incubation at room temperature for 2 hours → Anti His AF488 secondary antibody → DAPI staining, fluorescence microscopy imaging.

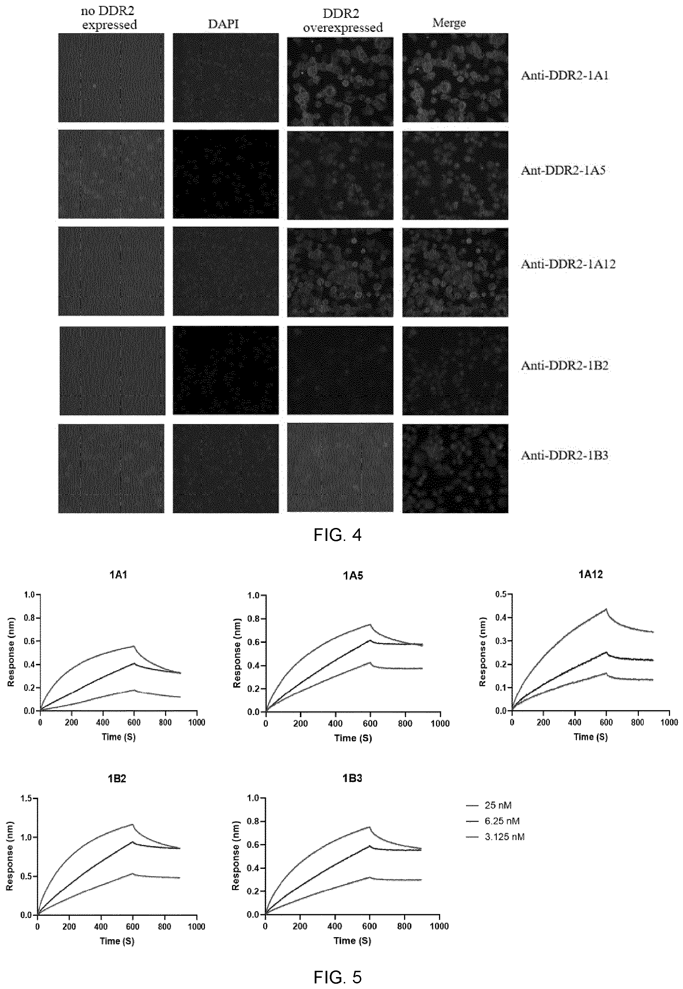

Result analysis: The analysis results of binding to DDR2 antigen are shown in Figure 4. It can be seen that the five nano-antibodies 1A1, 1A5, 1A12, 1B2, and 1B3 can all show binding activity to DDR2 antigen.

### Example 6: Affinity of DDR2 nano-antibodies to extracellular segment of DDR2 antigen

### Experimental procedure:

Five purified DDR2 nano-antibodies, namely 1A1, 1A5, 1A12, 1B2, and 1B3, were analyzed using the ForteBio biological layer interference (BLI) technique based on the molecular interaction analysis platform. The affinity between the antibodies and the antigen was determined using the BLI method, and the affinity between the nano-antibodies and the extracellular segment protein of DDR2 antigen was analyzed and detected.

Experimental consumables: Antibodies: dissolved in PBS (pH 7.4); Antigen: dissolved in PBS (pH 7.4); Sensor: Ni-NTA; Kinetics Buffer: PBST (PBS+0.02% Tween-20, pH 7.4); Regeneration Buffer: 10 mM Glycine-HCl, pH 1.7; Re-charged Buffer: 10 mM NiCl in H2O.

### Experimental protocol:

a. The probe was pre-wetted in a kinetics buffer for 10 minutes;
b. Baseline 1: Baseline the Biosensors in kinetics buffer for 180 s;
c. Fixation: the extracellular segment of antigen DDR2 with Hig tag was diluted using a kinetics buffer to 20 µg/ml, followed by sensor capturing; until 4 nM (300 s);
d. Baseline 2: Baseline the Biosensors in kinetics buffer for 60 s;
e. Binding: The antibody solution was diluted with a kinetics buffer to a certain concentration (from 100 nM, 2-fold dilution to 3.125 nM), and the sensor was inserted into the antibody solution for binding (600 s);
f. Dissociation: The sensor dissociated in the kinetics buffer (600 s);
g. Sensor regeneration: 10 mM Glycine HCl, pH 1.7 for 5 s;
h. Neutralization: After sensor regeneration, neutralization was performed in the kinetics buffer for 5 seconds;
i. Repeating step g for regeneration and step h for neutralization: for a total of 3 times (30 s);
j. Baseline 3: After regeneration of the sensor, it was added to 10 mM NiCl for 60 seconds.

Dynamic curves were prepared and relevant parameters were calculated. Binding dissociation curves with appropriate concentration gradients were selected and a 1:1 binding mode was used to fit all curves. Three curves with the best fit were chosen for graphical analysis, and finally important parameters, such as affinity values, binding constants, and dissociation constants, were obtained.

Result analysis: The analysis results of the binding of 5 purified DDR2 nano-antibodies: 1A1, 1A5, 1A12, 1B2, 1B3, to DDR2 antigen are shown in Figure 5.

**Table1: Affinity data of nano-antibodies**

| No. of the nano-antibody | Affinity |
|---|---|
| 1A1 | 1.22*10^-8 |
| 1A5 | 3.003*10^-9 |
| 1A12 | 4.359*10^-9 |
| 1B2 | 2.897*10^-9 |
| 1B3 | 2.902*10^-9 |

### Example 7: In vitro imaging results of DDR2 nano-antibodies

### Experimental procedure:

### (1) Labeling DDR2 nano-antibodies with indocyanine green (ICG)

Nb-DDR2 was dissolved in PBS at a concentration of 2 mg/mL followed by vortexing to mix uniformly.

ICG-NHS was dissolved in DMSO at a concentration of 2 mM followed by vortexing to mix uniformly.

500 µL of 2 mg/mL Nb-DDR2 solution was taken and transfered to a 1.5 mL centrifuge tube. 18 µL of 2 mM ICG-NHS solution was added to the Nb-DDR2 solution in 9 portions, 2 µL each time, with vortexing to mix uniformly for a few seconds each time.

The pH value of the mixed solution was detected, and the pH was adjusted to 8.5-9 using 2 M NaOH solution.

The centrifuge tube was placed on a 60 rpm shaker for reacting at room temperature for 2 hours.

A 0.5 mL ultrafiltration tube was used to centrifuge 14000 g for 10 minutes multiple times, unreacted ICG-NHS was removed, and the solution was replaced with 0.9% NaCl. The protein solution was filtered through a 0.22 µm filter membrane and stored at 4°C.

### (2) Imaging of mouse lung tissue slices

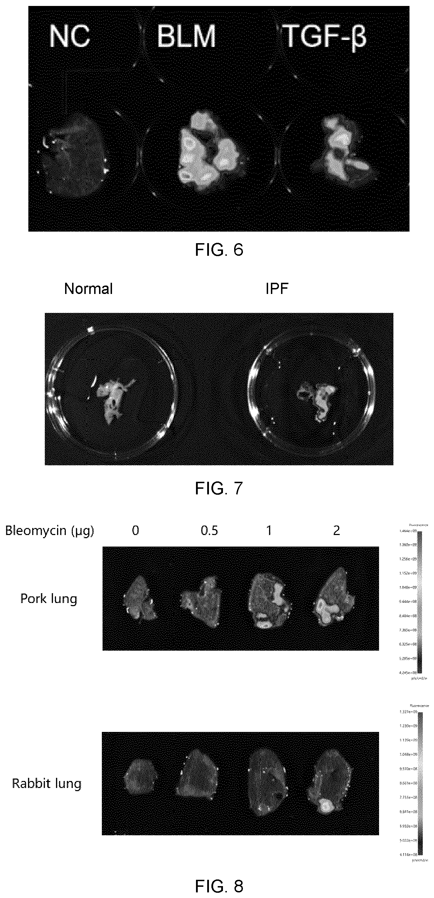

Fresh lung tissue from normal mice was cut into a thickness of 300 µm. One was used as an untreated control, one was induced with bleomycin, and the other one was induced with mTGF-β cytokines, both for 72 hours (both treatment groups were able to promote DDR2 expression). Then, each group was stained with ICG labeled DDR2 nano-antibody 1A12 (1 ug per sample) for 30 minutes, followed by washing multiple times with PBS to remove non-specific staining. Then, imaging was performed using a live imaging device. The imaging results are shown in Figure 6.

Result analysis: Purified DDR2 nano-antibodies labeled with ICG can specifically bind to tissues with high DDR2 expression in mice, which can be used for precise in vitro imaging.

### (3) Imaging of lung tissue slices in patients with idiopathic pulmonary fibrosis (IPF)

Lung tissues from normal donors and patients with idiopathic pulmonary fibrosis (IPF) were cut to a thickness of 600 µm in vitro, with high expression of DDR2 in IPF patients' lung tissues and low or no expression of DDR2 in normal donors' lungs. The Normal group was used as control, and lung tissue slices from IPF patients were used as the experimental group. After staining with ICG labeled DDR2 nano-antibody 1A12, in vitro live imaging was performed. The results are shown in Figure 7.

Result analysis: Purified DDR2 nano-antibodies labeled with ICG can specifically bind to tissues with high DDR2 expression in humans, which can be used for precise in vitro imaging.

### (4) Imaging of pig and rabbit lung tissue slices

Fresh lung tissues from normal pigs and rabbits were cut into thin slices with a thickness of 600 µm. Bolemycin was added at concentrations of 1 µg/mL, 2 µg/mL, and 4 µg/mL to induce the expression of DDR2 in the lung tissue. The lung tissue without adding of bleomycin was used as a blank control. After 72 hours of induction, 2 µg of ICG labeled DDR2 nano-antibody 1A12 was added, followed by incubating at 37 °C for 1 hour. The tissue slices were washed 5 times with PBS to remove unbound probes. Near infrared imaging was performed using a small animal live imaging device. The results are shown in Figure 8.

Result analysis: ICG labeled DDR2 nano-antibodies can specifically recognize highly expressed DDR2 in pig and rabbit lung tissues, which can be used for precise in vitro imaging.

### (5) Imaging of retinal neovascularization

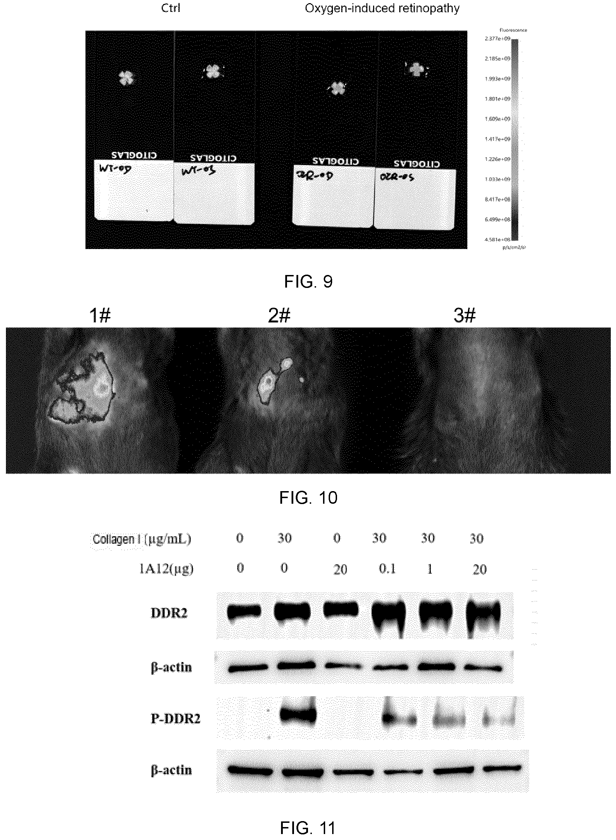

Smooth muscle cells in blood vessels are the main component of the vascular wall and can express DDR2. Retina samples were taken from normal mice and mice with oxygen induced lesions. After sectioning, 2 µg of ICG labeled DDR2 nano-antibody 1A12 was added, followed by incubating at room temperature for 1 hour. PBS was used to wash multiple times to remove unbound probes. Near-infrared imaging was performed using a small animal live imaging device. The results are shown in Figure 9.

Result analysis: ICG labeled DDR2 nano-antibodies can specifically recognize DDR2 in the mouse retina, demonstrating the distribution of blood vessels in the retina and oxygen induced retinal vascular lesions.

### Example 8: In vivo imaging results of DDR2 nano-antibodies

### Experimental procedure:

Bleomycin was administered at a dose of 1.7 mg/kg through endotracheal intubation into the lungs of C57 mice to establish a mouse model of pulmonary fibrosis. In the mouse model of pulmonary fibrosis, DDR2 was highly expressed in lung tissue. One week after modeling, 75 ug of ICG labeled 1A12 nano-antibody was injected through the tail vein, and in vivo imaging was performed. Mice 1 and 2 are pulmonary fibrosis models, while mouse 3 is a normal unmodeled mouse. Imaging and localization of pulmonary fibrosis sites in mice was performed using a small animal live imaging system.

The results show that the anti-DDR2 nano-antibodies provided by the present invention can achieve accurate imaging of pulmonary fibrosis lesions in mice, and can be used for precise in vivo diagnosis of early pulmonary fibrosis in the future, as shown in Figure 10.

### Example 9: Nano-antibody 1A12 inhibits collagen induced DDR2 protein phosphorylation (functional validation)

Experimental procedure: 293T cells overexpressing DDR2 antigen were seeded in a six well plate and allowed to grow to 70-80%. The cells were then starved by replacing with 0.75% FBS medium, which was replaced with a new 0.75% medium after 12 hours. Different amounts of DDR2 antibody 1A12 was added for incubattion. Collagen was added after 2 hours for stimulation, and the cells were lysed after 12 hours and protein was collected for Western blotting.

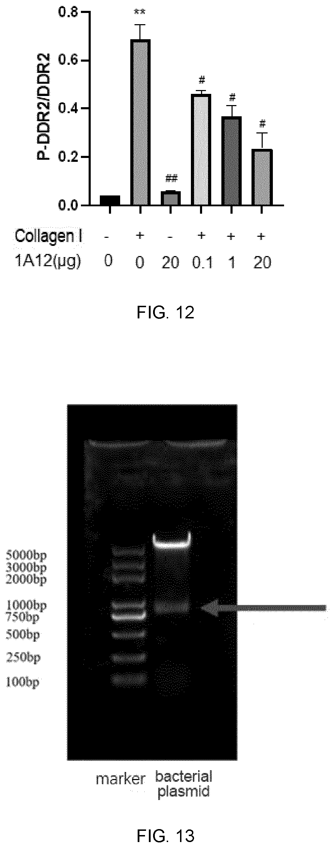

The experimental results are shown in Figures 11 and 12. The results showed that 1A12 could inhibit collagen induced phosphorylation of DDR2 protein in a dose-dependent manner.

### Example 10: Plasmid digestion identification of heterodimeric anti-DDR2 nano-antibody 1A1-1A12

In this example, heterodimeric nano-antibody 1A1-1A12 was constructed, which comprises the aforementioned nano-antibodies 1A1 and 1A12. The linker sequence is: GGGSGGGSGGGS (SEQ ID NO: 27), and the linking site is between the N-terminus of the sequence of 1A1 and the C-terminus of the sequence of 1A12. Characterization and experiments were performed on this antibody.

### Experimental procedure:

(1) Glycerol bacteria expressing DDR2 nano-antibody 1A1-1A12 was taken and shaken overnight. On the second day, small plasmid extraction was performed. 500 nanograms of plasmid was taken, followed by addition of 0.5 microliters of Ncol and Xhol rapid endonuclease, 1 microliter of 10x buffer. Double distilled water was added to achive a 10 microliter system, which was placed under 37°C for incubating for 30 minutes;
(2) 0.3 grams of agarose was weighed and placed in a triangle bottle, which was added with 30ml TAE buffer solution. The triangle bottle was heated in the microwave oven until the agarose was dissolved. When the agarose gel solution was cooled to about 65°C, ethidium bromide was added, and the mixture was fully mixed. The mold was fixed, with the comb placed, into which the agarose solution was poured and allowed to solidify;
(3) The sample and DNA marker were added into the wells, followed by being subjected to a 120 V electrophoresis condition for 20 minutes. The agar gel was taken for exposure development.

The results are shown in Figure 13. The results showed that the plasmid digestion size was correct and it was the target plasmid.

### Example 11: Coomassie blue staining of purified protein of heterodimeric anti-DDR2 nano-antibody 1A1-1A12

Experimental procedure: 2 micrograms of purified anti-DDR2 nano-antibody 1A1-1A12 was taken, and 5x protein Loading Buffer was added, followed by boiling and denaturing. The sample was loaded to an SDS-PAGE gel, and electrophoresis was performed for 1 hour under the condition of 220 volts. The electrophoresis was stopped, the SDS-PAGE gel was taken and placed in a proper amount of Coomassie blue dye solution. Staining was performed for 30 minutes at the speed of 60 rpm on the shaker, the dye was discarded, and double distilled water was used to wash for 2-3 times. Photos were taken under white light.

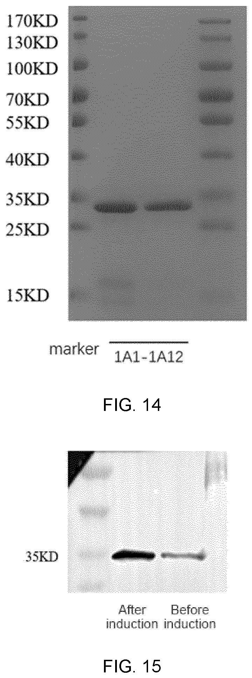

The results are shown in Figure 14. The results showed that the purified heterodimeric nano-antibody 1A1-1A12 had the correct size and no other impurities.

### Example 12: WB identification of heterodimeric anti-DDR2 nano-antibody 1A1-1A12

Experimental procedure: 2 micrograms of purified anti-DDR2 nano-antibody 1A1-1A12 was taken, and 5x protein Loading Buffer was added, followed by boiling and denaturing. The sample was loaded to an SDS-PAGE gel, and electrophoresis was performed for 1 hour under the condition of 220 volts. The electrophoresis was stopped, the SDS-PAGE gel was taken for membrane transfer, with the membrane transfer condition being 400 mA for 35 minutes. Blocking was performed with 5% skimmed milk powder for 1 hour, followed by incubating with Anti-His-HRP at room temperature for 1 hour. TBST was used to wash for three times, and development was performed.

The results are shown in Figure 15. The results showed that the purified heterodimeric nano-antibody 1A1-1A12 was of the correct size.

### Example 13: Flow cytometry identification of the binding activity of heterodimeric anti-DDR2 nano-antibody 1A1-1A12 to the antigen DDR2

Experimental procedure: DDR2 293T and 293T cells were transfered to a 1.5 mL centrifuge tube, and 1 µg of anti-DDR2 nano-antibody 1A1-1A12 was added for incubating for 1 hour. Centrifugation was performed at 300 g for 5 minutes, and the supernatant was discarded and PBS was added. Washing was perfomed for 3 times, and anti-His-488 secondary antibody was added for incubating in the dark for 1 hour, followed by washing 3 times with PBS, and flow cytometry detection was performed.

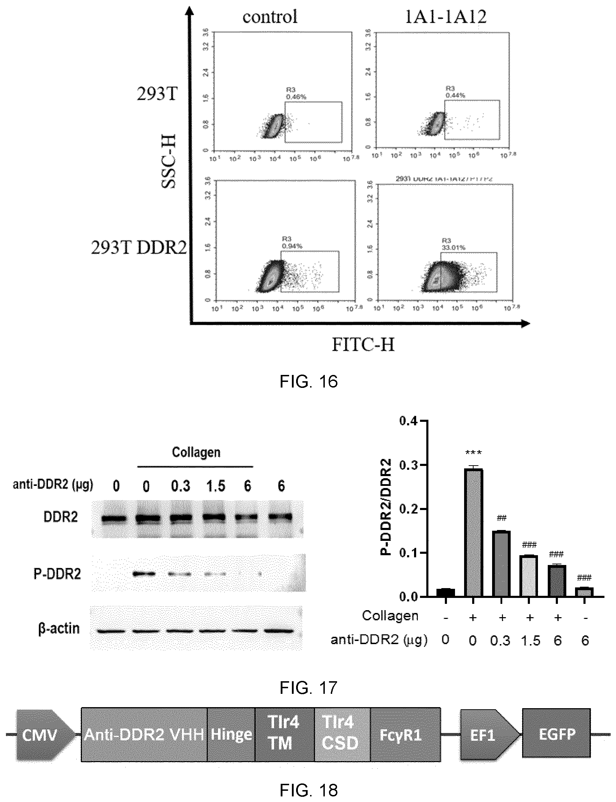

The results are shown in Figure 16. The results showed that the purified heterodimeric nano-antibody 1A1-1A12 has the ability to bind to the corresponding antigen DDR2.

### Example 14: Heterodimeric anti-DDR2 nano-antibody 1A1-1A12 inhibits collagen induced DDR2 protein phosphorylation (functional validation)

### Experimental procedure:

(1) DDR2-293T cells were seeded in a six well plate, with one well per group, allowing the cell density to reach 85%;
(2) The cells were starved for 12 hours by replacing with 0.75% fetal bovine serum medium. Different concentrations of antibody 1A1-1A12 solutions were prepared using the same starving medium;
(3) The supernatant of the starving medium was discarded, followed by replacing with different concentrations of antibody medium for incubating for 2 hours;
(4) Collagen was added at a concentration of 30 micrograms/milliliter for stimulation, and after 12 hours of stimulation, the cells were lysed and protein was extracted;
(5) WB experiment was performed to detect the phosphorylation level of DDR2 protein in each group of cells.

The results are shown in Figure 17. The results showed that heterodimeric nano-antibody 1A1-1A12 had inhibitory effects when bound to DDR2 antigen.

### Example 15: Chimeric antigen receptor

A CAR molecular vector was constructed with the DDR2 nano-antibody 1A12 sequence (as shown in Figure 18), adenovirus were packaged, mouse primary macrophages were infected, and αDDR2 CAR-M cells were obtained and used for the following experiments.

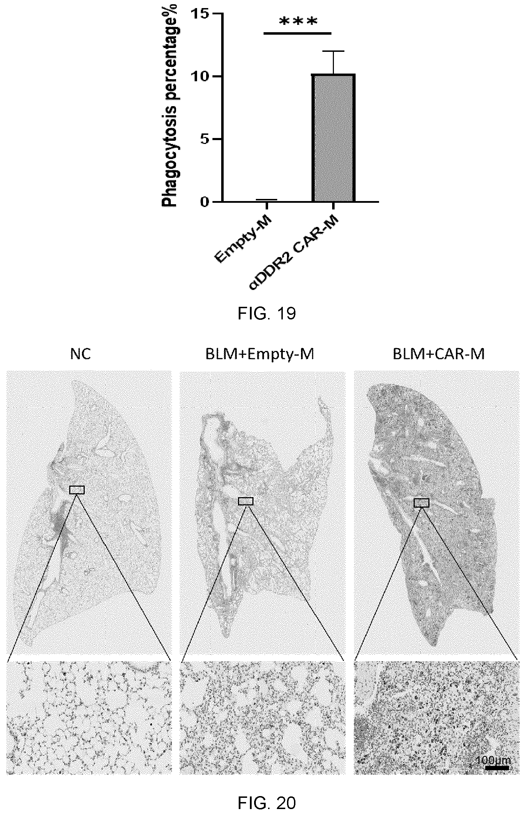

Empty-M (uninfected macrophages) and αDDR2 CAR-M were co cultured with 293T-DDR2 cells in a 1:1 ratio. The percentage of FITC and APC double positivity was used as the phagocytic percentage of 293T-DDR2, and statistical analysis was performed, and the results were expressed as Mean±SEM. As shown in Figure 19, the difference between Empty-M and CAR-M was statistically significant. It can be seen that CAR-M cells constructed with 1A12 engulf DDR2 positive 293T cells at a significantly high rate.

Empty-M and αDDR2 CAR-M were injected into the tail vein of mice with a left lung model induced by bleomycin, with 5X10⁵ each. After 15 days, the mice were euthanized and their left lungs were taken. Normal mice without macrophage injection were used as control, and CD68 immunohistochemistry was performed. The results are shown in Figure 20. It can be seen that CD68 immunohistochemistry showed brown fine granules in lung sections of all groups of mice. Weak positive expression of CD68 was observed in lung tissue sections of the NC group and BLM+Empty-M group, mainly distributed around the airway and in the alveolar septum; The positive expression of CD68 was significantly enhanced and evenly distributed in the BLM+CAR-M group. Based on the above results, it is considered that the adoptive transfer of αDDR2 CAR-M can locate to the area of high DDR2 expression - the left lung of mice. It indicats that CAR-M cells constructed with 1A12 were concentrated in tissues with high DDR2 expression in mice.

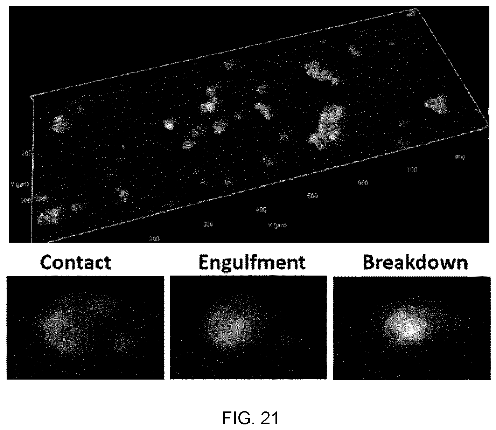

The targeted phagocytic effect of αDDR2 CAR-M on 293T-DDR2 was verified using a lattice light plate super-resolution microscopy. αDDR2 CAR-M were themselves with green fluorescence. Dil (an orange red fluorescent dye) was used to dye 293T-DDR2 red. The two were mixed in a 1:1 ratio and loaded, followed by taking pictures continuously for 16 hours, once every 3 minutes. The results are shown in Figure 21. Green (CAR-M) and red (293T-DDR2) cells can be seen, among which yellow cells are considered as formed by green CAR-M cells fully engulfing the red 293T-DDR2, after which the red dye carried by 293T-DDR2 entered into the CAR-M. It proves that CAR-M cells constructed with 1A12 can effectively target and engulf DDR2 positive 293T cells.

0.75mg/ml 40 µl volume of bleomycin (BLM) was injected into the trachea of each group of mice. On the 8th day, Micro-CT was performed to screen mice with left lung modeled, which was divided into groups according to the degree of lesion. On the 10th day, PBS, Empty-M, and αDDR2 CAR-M were respectively administered via tail vein injection. On the 26th day, Micro-CT was performed again to evaluate the treatment effect. On the 28th day, the mice were euthanized and their left lung were taken and fixed.

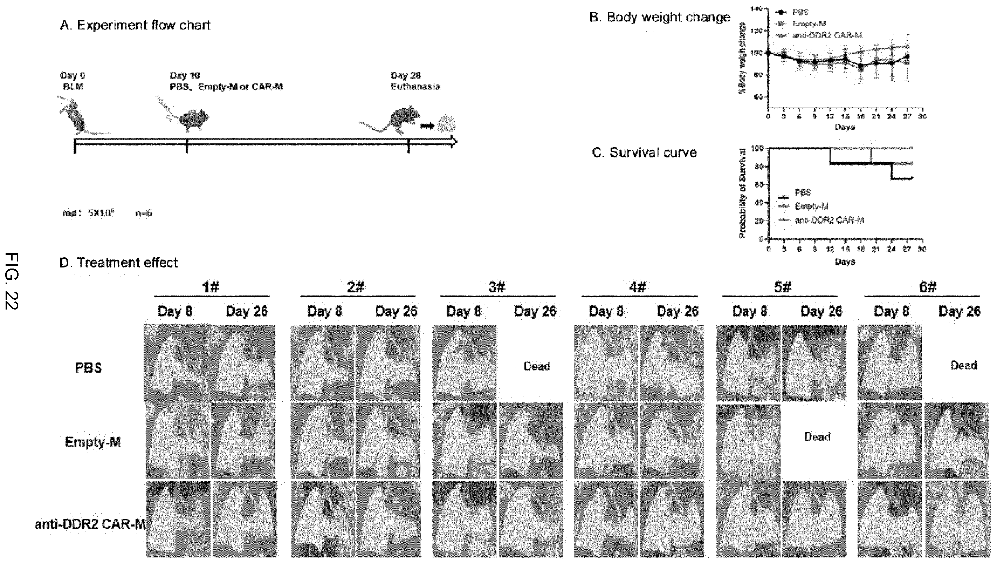

As shown in Figure 22, Figure A is the experimental flowchart, Figure B is the body weight change chart of mice weighed every 3 days after BLM modeling, Figure C is the survival curve chart of each group of mice from the day of BLM modeling to the 28th day after modeling, and Figure D is the CT 3D images obtained by lung Micro-CT taken on the 8th and 26th days after modeling for each group of mice.

From the perspective of weight, the CAR-M group of mice showed a trend of first decreasing and then increasing in weight. On the 28th day, they were able to recover to their original weight level, while the other two groups showed some weight recovery but could not return to their original weight. From the survival curve, there was no mouse death in the CAR-M group, while the other two groups showed mouse death, with the PBS group being even more severe. From CT, most of the lung lesions in the CAR-M group of mice have recovered compared to day 8, while the other two groups have not shown significant recovery or even worsened. In summary, compared with the control group, mice injected with αDDR2 CAR-M had significantly higher body weight, significantly increased survival rate (no deaths until day 28), controlled lesion severity, which showed significant therapeutic effects.

### Example 16: Procedure for labeling nano-antibody 1A12 with conventional isotopes such as ⁶⁴Cu and ⁶⁸Ga

### 1. Coupling with NOTA

(1) 12000 g centrifugation was performed on the antibody solution, the supernatant was taken, and the solvent of the the antibody solution was replaced with 0.1 M ammonium acetate (pH=7), and after replacement, the concentration of nano-antibody 1A12 cys was adjusted to 1.33 mg/mL. The concentrations before and after replacement were measured, and the concentration after replacement was used as the standard.
(2) 50 mM Mal-NOTA (dissolved in DMSO) was taken, and the molar ratio of antibody to Mal-NOTA was adjusted to be 1:5. Mal-NOTA was diluted with DMSO to a volume less than 10% of the reaction system volume (for example, if the reaction system was 1 mL, then the Mal-NOTA was diluted to 80-90 µL, with shaking to fully dissolve Mal-NOTA in DMSO). The Mal-NOTA solution was added in portions to the antibody solution, 10 µL/time, with shaking for tens of seconds after each addition. The pH of the solution was adjusted to 7 (the reaction solvent was 0.1 M ammonium acetate, pH did not need to be adjusted, confirmation by detecting was sufficient). It was placed on a shaker for reacting at room temperature for 2 hours, with the rotating speed being 150 rpm.
(3) After the reaction was completed, 12000 g centrifugation was performed on the antibody solution. The supernatant was taken, and the antibody solution was purified and concentrated with 0.1 M ammonium acetate, with every 100 µg of antibody concentrated to 50-80 µL. A sealing film was used to seal the opening, and it was stored at -80°C in a refrigerator.

### 2. Labeling with conventional isotopes such as ⁶⁸Ga and ⁶⁴Cu

(1) ⁶⁸Ga or ⁶⁴Cu or other isotopes was taken and added to the precursor solution (⁶⁴Cu should be concentrated as much as possible, for 100 µg precursor the activity in the feeding was about 1.5 mCi, and the final reaction volume did not exceed 150 µL), followed by mixing uniformly, and adjusting the pH value to 4-5 (usually no adjustment was needed, the pH would be 4-5 after mixing).

If there was a shaking temperature controller, it was placed in the shaking temperature controller at 37°C for reacting for 2 hours. If not, it was heated at 37°C for reacting for 2 hours (water bath, oven, etc.).

(3) Chromatography paper was used for application of sample, followed by developing with a developing agent, and detecting the labeling rate using iTLC (the developing agent was sodium citrate).

### Example 17: Imaging with isotope ⁶⁸Ga labeled nano-antibody 1A12

⁶⁸Ga labeled nano-antibody 1A12 was prepared according to the procedure described in Example 16.

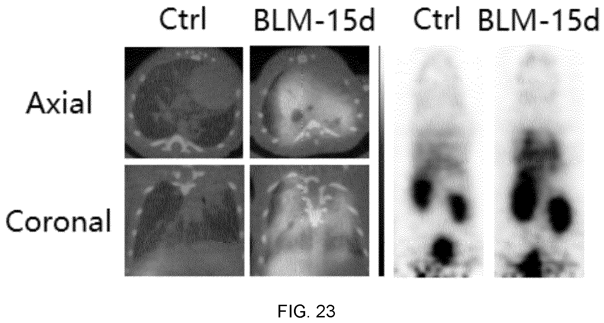

Mice modeled with bleomycin (BLM) for 15 days (BLM-15d) and normal control mice (Ctrl) were taken and administered via the tail vein respectively with ⁶⁸Ga labeled nano-antibody 1A12 at a concentration of 200 µCi per mouse, to perform PET-CT imaging. The imaging results are shown in Figure 23. The results showed that mice modeled with BLM exhibited significant uptake in the lungs compared to normal mice. It shows that nano-antibody conjugated with isotopes can be used for PET-CT imaging.

It should be understood that although the present invention has been specifically disclosed through preferred embodiments and optional features, those skilled in the art may modify, improve, and vary the present invention disclosed herein, and these modifications, improvements, and variations are considered within the scope of the present invention. The materials, methods, and embodiments provided herein are representative and exemplary of preferred embodiments and are not intended to limit the scope of the invention.

## Claims

1. A nano-antibody binding to DDR2, wherein the nano-antibody comprises a CDR1, a CDR2, and a CDR3 comprising or being the CDR1, CDR2, and CDR3 of a nano-antibody selected from the amino acid sequence of any one of SEQ ID NO: 4, SEQ ID NO: 8, SEQ ID NO: 12, SEQ ID NO: 16, and SEQ ID NO: 20, or an equivalent variant of each thereof.

2. The nano-antibody according to claim 1, wherein the CDR1, CDR1, and CDR3 are defined based on any one of the IMGT, Kabat, Chothia, Contact, or Martin definition scheme.

3. The nano-antibody according to claim 1, wherein:
(a) the CDR1 comprises or is the sequence set forth in SEQ ID NO: 1 or an equivalent variant thereof, the CDR2 comprises or is the sequence set forth in SEQ ID NO: 2 or an equivalent variant thereof, the CDR3 comprises or is the sequence set forth in SEQ ID NO: 3 or an equivalent variant thereof;
(b) the CDR1 comprises or is the sequence set forth in SEQ ID NO: 5 or an equivalent variant thereof, the CDR2 comprises or is the sequence set forth in SEQ ID NO: 6 or an equivalent variant thereof, the CDR3 comprises or is the sequence set forth in SEQ ID NO: 7 or an equivalent variant thereof;
(c) the CDR1 comprises or is the sequence set forth in SEQ ID NO: 9 or an equivalent variant thereof, the CDR2 comprises or is the sequence set forth in SEQ ID NO: 10 or an equivalent variant thereof, the CDR3 comprises or is the sequence set forth in SEQ ID NO: 11 or an equivalent variant thereof;
(d) the CDR1 comprises or is the sequence set forth in SEQ ID NO: 13 or an equivalent variant thereof, the CDR2 comprises or is the sequence set forth in SEQ ID NO: 14 or an equivalent variant thereof, the CDR3 comprises or is the sequence set forth in SEQ ID NO: 15 or an equivalent variant thereof; or
(e) the CDR1 comprises or is the sequence set forth in SEQ ID NO: 17 or an equivalent variant thereof, the CDR2 comprises or is the sequence set forth in SEQ ID NO: 18 or an equivalent variant thereof, the CDR3 comprises or is the sequence set forth in SEQ ID NO: 19 or an equivalent variant thereof.

4. The nano-antibody according to claim 1, wherein the nano-antibody is a humanized nano-antibody.

5. The nano-antibody according to claim 1, wherein the nano-antibody comprises or is the sequence selected from any one of SEQ ID NO: 4, SEQ ID NO: 8, SEQ ID NO: 12, SEQ ID NO: 16, and SEQ ID NO: 20, and an equivalent variant of each thereof.

6. A polynucleotide comprising a polynucleotide encoding the nano-antibody according to any one of claims 1 to 5.

7. The polynucleotide according to claim 6, wherein the polynucleotide comprises or is the sequence selected from any one of SEQ ID NO: 21, SEQ ID NO: 22, SEQ ID NO: 23, SEQ ID NO: 24, and SEQ ID NO: 25, and an equivalent variant of each thereof.

8. A vector comprising the polynucleotide of claim 6 or 7.

9. A non-human host cell, comprising the vector of claim 8.

10. A chimeric antigen receptor, comprising an extracellular domain capable of binding to an antigen, a transmembrane domain, and an intracellular domain, wherein the extracellular domain capable of binding to an antigen comprises the nano-antibody according to any one of claims 1 to 5.

11. The chimeric antigen receptor according to claim 10, wherein the transmembrane domain comprises a Tlr4 transmembrane region, and the intracellular domain comprises a Tlr4 CSD region and an FcγR I intracellular functional region.

12. A modified immune cell expressing the chimeric antigen receptor of claim 10 or 11.

13. The immune cell according to claim 12, wherein the immune cell is a macrophage.

14. A bispecific antibody, comprising a first binding moiety binding to a first antigen and a second binding moiety binding to a second antigen, wherein the first binding moiety comprises the nano-antibody according to any one of claims 1 to 5.

15. The bispecific antibody according to claim 14, wherein the second binding moiety comprises the nano-antibody according to any one of claims 1 to 5.

16. The bispecific antibody according to claim 15, wherein the nano-antibody comprised in the first binding moiety is different from the nano-antibody comprised in the second binding moiety.

17. An antibody-drug conjugate, comprising an antibody targeting DDR2, a drug, and a linker connecting the antibody and the drug, wherein the antibody targeting DDR2 comprises or is the nano-antibody according to any one of claims 1 to 5.

18. An siRNA conjugate targeting a DDR2 positive cell, comprising: (i) an antibody targeting DDR2, which comprises or is the nano-antibody according to any one of claims 1 to 5, (ii) an siRNA that inhibits the expression of a survival gene in a DDR2 positive cell, and (iii) a linker positioned between the nano-antibody and the siRNA.

19. A reagent for imaging and/or diagnosis, comprising the nano-antibody according to any one of claims 1 to 5 and a detectable label connected with the nano-antibody.

20. The reagent according to claim 19, wherein the detectable label is a fluorescent label or a radioisotope.
